(19) Europäisches Patentamt / European Patent Office / Office européen des brevets

(11) EP 3 403 994 A1

(12) **EUROPEAN PATENT APPLICATION**

(43) Date of publication:
**21.11.2018 Bulletin 2018/47**

(21) Application number: **17305576.5**

(22) Date of filing: **18.05.2017**

(51) Int Cl.:
*C01B 32/00* (2017.01) *B82Y 30/00* (2011.01)
*B82Y 40/00* (2011.01) *H01B 1/04* (2006.01)
*C01B 32/184* (2017.01) *C01B 32/194* (2017.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **CENTRE NATIONAL DE LA RECHERCHE SCIENTIFIQUE**
  **75016 Paris (FR)**
- **Alma Mater Studiorum - Università di Bologna**
  **40126 Bologna (IT)**

(72) Inventors:
- **HOF, Ferdinand**
  **33600 Pessac (FR)**
- **PÉNICAUD, Alain**
  **33000 Bordeaux (FR)**
- **BONI, Alessandro**
  **42123 Reggio Emilia (IT)**

(74) Representative: **Novagraaf Technologies**
**Bâtiment O2**
**2, rue Sarah Bernhardt**
**CS90017**
**92665 Asnières-sur-Seine Cedex (FR)**

(54) **GRAPHENE-SUPPORTED METAL AND/OR METAL OXIDE NANOPARTICLE COMPOSITES, METHOD FOR MAKING SAME AND USES THEREOF**

(57) The present invention relates to a nanographene-supported metal and/or metal oxide nanoparticle composite, and method for preparing same, and uses thereof.

Specifically, the present invention relates to the uses of nanographene-supported metal and/or metal oxide nanoparticle composite as catalytic material, in electrocatalysis, as catalyst for gas-phase chemical reactions, as slurry catalyst for liquid-phase chemical reactions, for water purification and waste water treatment, in the treatment of cancerous tumors, and/or in Magnetic Resonance Imagery.

The present invention finds applications in the industrial technical field, in particular in the material, chemical and pharmaceutical technical fields.

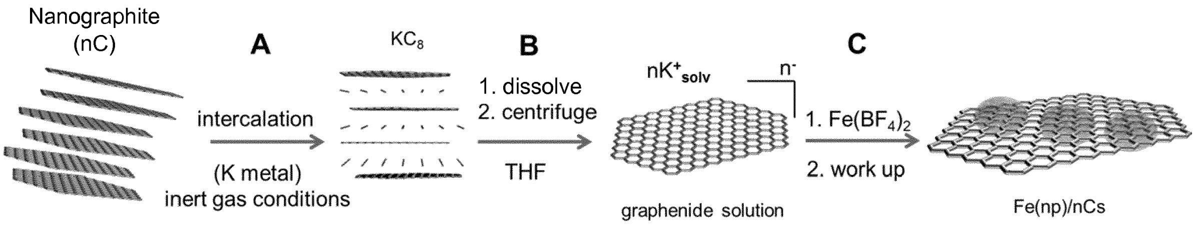


**FIGURE 1**

EP 3 403 994 A1

## Description

### TECHNICAL FIELD

The present invention relates to a nanographene-supported metal and/or metal oxide nanoparticle composite, and method for preparing same, and uses thereof.

Specifically, the present invention relates to the uses of nanographene-supported metal and/or metal oxide nanoparticle composite as catalytic material, in electrocatalysis, as catalyst for gas-phase chemical reactions, as slurry catalyst for liquid-phase chemical reactions, for water purification and waste water treatment, in the treatment of cancerous tumors, and/or in Magnetic Resonance Imagery.

The present invention finds applications in the industrial technical field, in particular in the material, chemical and pharmaceutical technical fields.

In the description that follows, the references between square brackets ([ ]) refer to the list of references that is provided after the Examples.

### BACKGROUND

Graphene has received huge attention over the past decade from various fields due to its remarkable characteristics, notably its excellent electronic mobility, thermal conductivity, mechanical strength, low charge transfer energy combined with its chemical stability.

Graphene finds numerous applications in various fields such as automobile, energy, aerospace, construction, electronic products, medicine, military, and communication, as a next generation energy storage material, semiconductor material alternative to silicon, super capacitor, light weight structural parts, electromagnetic shielding material, sensor, display, and the like.

Meanwhile, metal nanoparticles and metal oxide nanoparticles are being used in various industrial fields such as electronic materials, catalysts, sensors, inks, and secondary battery electrode materials based on their optical, electrical, magnetic, and chemical characteristics which may vary depending on the particle size.

Recently, research efforts have focused on combining the properties of graphene and metal nanoparticles and metal oxide nanoparticles by producing metal oxide nanoparticle/graphene and metal nanoparticle/graphene composites.

Currently, the synthesis of nano composite materials containing graphene sheets and nanoparticles are based on either graphene oxide / reduced graphene oxide (GO / rGO) systems or on aqueous dispersions of few layer graphene. In order to generate composite materials with nanoparticles, the carbon framework, metal salts and reduction agent needs to be merged. However, some major issues are: lack of control over size and particle density; side reaction of the reduction agent with the surfactant or functional groups as well as trapping thereof in-between the sheets. Consequently, the performances as an electro catalyst are drastically impacted. Surprisingly, there are only few examples published, exploiting the reducing potential of GICs in order to generate metal graphite composite materials. The characterization of the obtained composite materials has been even less investigated. In addition, current methods are confronted to problems with the size distribution of the metal oxide/metal particles formed on the surface of the graphene composite which tends to be too broad, and it is difficult to reduce the size to nanometers, thereby deteriorating the overall performance of the composite.

Therefore, there remains a dire need to develop methods for preparing metal oxide nanoparticle/graphene and metal nanoparticle/graphene composites that overcome the above drawbacks, notably in terms of metal/metal oxide particle size and overall performance of the resulting composite.

### DETAILED DESCRIPTION OF CERTAIN EXEMPLARY EMBODIMENTS OF THE INVENTION

In a first aspect, the present invention specifically aims at meeting such need by providing a method for preparing nanographene-supported metal and/or metal oxide nanoparticle composites, comprising the following steps:

a) dissolving a nanographite intercalation compound in an aprotic organic solvent (A) or a mixture (A') of aprotic organic solvents under anhydrous inert atmosphere, preferably in the absence of sonication; thereby leading to an organic nanographenide solution;

b) reacting the organic nanographenide solution obtained in step a) with a suitable amount of at least one metal salt and/or at least one metal complex or a mixture of at least two metal salts and/or metal complexes under anhydrous inert atmosphere; thereby leading to a suspension of nanographene-supported metal nanoparticles; and

c) optionally oxidizing the metal nanoparticles present on the nanographene support;

wherein the process is free of reducing agent other than the nanographenide formed in step a).

As used herein, the term "nanographite" refers to carbon nanoparticles having a lateral size in the nm range, typically between 1 and 900 nm, preferentially between 1 and 200 nm, between 1 and 100 nm, preferentially between 1 and 25 nm; sharing or resembling the crystal structure of graphite, consisting of planes of carbon atoms arranged in planar hexagonal arrays, with the carbon atom planes stacked on top of each other (delocalized $\pi$ bonding between the carbon atom sheets). A nanographite particle contains several nanographene layers packed on top of each other between 11 and an infinite number of layers and a lateral size in the nanometer range, as defined above. Nanographite particles may exhibit any polytype of natural graphite, including lubricostratic or turbostratic packing of carbon sheets.

Throughout the document, the term "lateral size", whether referring to nanographite or nanographene particles, refers to the largest dimension of the planar carbon atom sheet surface of the nanographite or nanographene particles. The term is not to be confused with the thickness of the nanographite or nanographene particles, which refers to the height of the stacked carbon atom planes making up the nanographite or nanographene particles.

As used herein, the term "graphene", takes the conventional meaning often used in the modern scientific literature, and refers to graphitic material composed of a few layers, for example 1 to 10 layers, preferably 1 to 5 layers, preferably 1 to 3 layers of carbon atoms sheets. Graphene thickness can be calculated from the number of layers multiplied by 0.35 nm.

As used herein, the term "nanographene" refers to graphene, as defined above, having a lateral size in the nanometer range; typically between 1 and 900 nm, preferentially between 1 and 200 nm, preferentially between 1 and 100 nm, preferentially between 1 and 25 nm.

Graphite is known for leading to intercalation compounds (graphite intercalation compounds or GIC) with either electron donors or acceptors. In an analogous manner as GICs, as used herein, the term "nanographite intercalation compound" refers to nanographite as defined above, intercalated with atomic or molecular species, which may be electron donors or acceptors. For example, the term refers to a compound comprising at least two individual negatively or positively charged nanographene planes and intercalated by positive or negative counter ions. Nanographite alkali salts are a particular example of nanographite intercalation compound where the nanographene planes are negatively charged and the counter-ions are alkali ions. The nanographite intercalation compound can be in the form of a binary compound having the formula $MC_x$ where M represents a positive counter-ion of an alkali metal ($M^+$), and x represents an integer between 6 and 200. More particularly, the alkali metal can be potassium. For example, the nanographite intercalation compound can be a binary compound having the formula $KC_8$.

As used herein, for example in reference to the nanographene-supported composite of the invention, as well as steps b) and c) of the process of the invention, the term "metal nanoparticle" or "metal oxide nanoparticle" refers to metal or metal oxide particles of roughly spheroidal shape and size, i.e. whose diameter is in the nanometer range. For example, the metal or metal oxide nanoparticles may have a diameter from 0.5 to 100 nm, preferably from 0.5 to 20 nm, preferably from 0.5 to 15 nm, preferably from 0.5 to 10 nm, preferably from 0.5 to 5 nm. Additionally, throughout the document, the term "metal nanoparticle" encompasses nanoparticles of pure metal, and nanoparticles of metal alloy (in other words, "metal" in the expression "metal nanoparticle" refers to a pure metal (for example Fe, Ni, Pt, Rh, Mn, Cu and/or Co) or a metal alloy (for example Fe/Pt, Fe/Rh, Co/Pt or Co/Rh alloy). Likewise, throughout the document, the term "metal oxide nanoparticle" encompasses nanoparticles of pure metal oxide, and nanoparticles of metal alloy oxide.

As used herein, the expression "aprotic organic solvents" refers to any aprotic organic solvent known from one skilled in the art adapted for dissolving a nanographite intercalation compound. It may be for example a polar organic solvent. Advantageously, a single aprotic organic solvent may be used in step a) (i.e., aprotic organic solvent (A)).

When an organic solvent mixture (A') is used in step a), it may be a binary, ternary, or higher-order solvent mixture (i.e., mixture or two, three, or more aprotic organic solvents). Preferably, (A') may be a binary or ternary, most preferably binary organic solvent mixture.

Advantageously, the aprotic organic solvent(s) used in step a) may be polar. As used herein, "polar" when referring to organic solvents, including the aprotic organic solvent(s) of step a) refers to any organic solvent having a dielectric constant e $\geq$4.

Throughout the present document, unless mentioned otherwise, all dielectric constant values (e) are provided with reference to a temperature of 25°C. In other words, unless mentioned otherwise, the expression "dielectric constant $\varepsilon \geq n$" means "dielectric constant $\varepsilon \geq n$ at 25°C".

For example, the aprotic organic solvent (A) and the aprotic organic solvents in organic solvent mixture (A') may have a dielectric constant e $\geq$4, e $\geq$5, $\geq$6, $\geq$7, $\geq$8, $\geq$9 or $\geq$10 at 25°C. Exemplary polar aprotic organic solvents that may be used in step a), as single solvent (A), include, but are not limited to, ethers such as tetrahydrofuran (THF), methyl-THF (Me-THF), dimethoxyethane (DME), methyl tert-butyl ether (MTBE), diethyl ether, or CycloPentylMethylEther (CPME), dimethylsulfoxide (DMSO), N-methylpyrrolidone (NMP), dimethylformamide (DMF), N-methylformamide (NMF), sulfolane, acetonitrile, nitromethane, ethylacetate, 2-butanone, or dimethylacetamide (DMA). When a mixture (A') is used as solvent system, it may comprise a mixture of two or more of the above-mentioned solvents.

Preferably the polar aprotic organic solvents that may be used in step a), as single solvent (A) or mixture (A'),

may be an ether, for example THF, DMA, DMF, preferably THF. Preferably, a single polar solvent (A) may be used in step a) selected from an ether (such as THF), DMA or DMF, most preferably THF.

As used herein, the expression "anhydrous inert atmosphere" refers to a gas or a gaseous mixture which does not favour oxidation of dissolved nanographite intercalation compound and/or nanographene-supported metal nanoparticles. For example, step a) and/or b), preferably both steps a) and b), may be carried out under an oxygen-free and moisture-free atmosphere. Advantageously, step a) and/or b), preferably both steps a) and b), may be carried out under an argon, helium or nitrogen gas atmosphere.

## Nanographite

The nanographite intercalation compound may be prepared using any source of graphite particles having a lateral size in the nanometer range, typically between 1 and 900 nm, preferentially between 1 and 100 nm, preferentially between 1 and 25 nm.

For example, the source of graphite particles having a lateral size in the nanometer range, may be prepared from sonication of graphite. Any type of graphite that can be intercalated may be used for that purpose, including disordered graphite, crystalline flake graphite, natural and lump graphite, highly ordered pyrolytic graphite (HOPG), graphitic nanofibres, and graphitic nanoplatelets. The graphite may first be coarsely crushed into a powder, and then sonicated, to yield particles of graphite having a lateral size in the nanometer range, as defined herein (i.e., nanographite).

Alternatively, the source of nanographite (graphite particles having a lateral size in the nanometer range, as defined herein), may be prepared from splitting a methane/carbon dioxide gaseous mixture, as starting carbon source material (splitting $CH_4/CO_2$ yields C and $H_2$). The process for splitting $CH_4/CO_2$ mixture may be any $CH_4/CO_2$ splitting process known from the skilled person. For example, the reader may refer to the teachings of Hof et al. "Conductive inks of graphitic nanoparticles from a sustainable carbon feedstock" Carbon 111 (2017) 142-149 **[1]**, which involves microwave plasma technology. The $CH_4/CO_2$ mixture may be prepared from any available source of $CH_4$ and $CO_2$, be it synthetic, natural, from biomass or organic waste. Advantageously, the nanographite obtained from such $CH_4/CO_2$ splitting process may exhibit turbostratic packing of the carbon sheets.

As part of the preparation method of the nanographite source material, a subsequent purification step may be included. For example, raw nanographite material (e.g., obtained from sonication of graphite particles or microwave plasma cracking of $CH_4/CO_2$) may be subjected to a heat treatment process. Specifically, raw nanographite material may be annealed at a temperature below the decomposition temperature of the material (<600°C), for example at 500°C ± 20°C. The heat treatment may incur 10-30% loss of carbon weight, corresponding to the loss of amorphous carbonaceous material and other impurities in the raw nanographitic material. Such purification step allows to obtain a more homogeneous nanographite material, with a calibrated size distribution both in thickness (number of graphene layers) and in lateral size of the nanographite particles. Typically, after heat treatment purification, the nanographite particles may exhibit (i) a lateral size distribution of 30-80 nm with a mean value ca 40 nm ± 5 nm, and (ii) a thickness corresponding to 20-30 graphene layers. Such purified nanographite may be advantageously used in the process of the present invention. It may additionally exhibit turbostratic packing of the carbon sheets.

## Nanographite intercalation compound

The generation of the nanographite intercalation compound can be carried out by any method known in the field of graphite intercalation compounds. For example, a nanographite intercalation compound may be prepared by reduction of the corresponding nanographite material. Exemplary methods include reduction of nanographite by an alkali metal $M_0$ in vapour phase or liquid phase; electrochemical reduction of a nanographite material; and reduction of a nanographite by an alkali metal salt of formula $M_0^+B^-$, wherein $M_0^+$ represents an alkali metal cation and $B^-$ represents an anion of an organic radical. In each of the above methods, the nanographite may have a lateral size between 1 and 900 nm, preferentially between 1 and 200 nm, preferentially between 1 and 100 nm, preferentially between 1 and 25 nm. Advantageously, in each of the above methods, the nanographite may first be subjected to a heat treatment purification step, as described above. As such, in each of the above methods, the nanographite may advantageously exhibit (i) a lateral size distribution of 30-80 nm with a mean value ca 40 nm ± 5 nm, and (ii) a thickness corresponding to 20-30 graphene layers. When the reduction involves an alkali metal $M_0$ or an alkali metal salt $M_0^+B^-$, the alkali metal $M_0$ can be any alkali metal allowing the implementation of the present invention. It can be selected for example from lithium, sodium, potassium, rubidium or cesium. Preferably, the alkali metal $M_0$ may be lithium, sodium or potassium. For example, a nanographite intercalation compound may be prepared by reduction of a nanographite material directly in the presence of an alkali metal $M_0$, such as Na, Li, K, Rb or Cs, preferably Na, Li or K. Thus, the reduction can be carried out directly in the presence of an alkali metal $M_0$, for example in liquid or vapour phase or in ammonia. Reduction methods in the presence of an alkali metal are well known in the art, and may be applied or adapted to graphite nanocarbon obtained from sonication of graphite or from splitting of $CH_4/CO_2$ gaseous mixtures. The person skilled in the art will know how

to identify appropriate experimental conditions for implementing a reduction method in the presence of an alkali metal $M_0$, for example in vapour phase or in ammonia. For example, nanographite intercalation compounds can be prepared by reacting nanographite and K between 70 and 200 °C (K is liquid above 65 °C) under argon atmosphere. In addition, the person skilled in the art can adapt and apply the teachings described in "Synthesis of graphite intercalation compounds", A. Herold in Chemical physics of intercalation, A.P. Legrand and S. Flandrois Eds, NATO ASI Series, series B, Vol. 172, pp. 3-45 (1987). **[2]**

Alternatively, the reduction of the nanographite material may also be carried out in the presence of an alkali metal salt obtained from an alkali metal. For example, the nanographite intercalation compound may be prepared by the addition of a polyaryl alkali salt having the formula $M_0^+B^-$ to a nanographite material under inert atmosphere, wherein:

$M_0^+$ represents a cation of an alkali ion, and

$B^-$ represents an anion of a polyaromatic compound.

Such polyaryl alkali salts and methods for preparing them are described for example in WO 2009/056696 **[3]**; C. Stein, J. Poulenard, L. Bonnetain, J. Golé, C.R. Acad. Sci. Paris 260, 4503 (1965) **[4]**; "Synthesis of graphite intercalation compounds", A. Herold in Chemical physics of intercalation, A.P. Legrand and S. Flandrois, Eds, NATO ASI Series, series B, Vol. 172, pp. 3-45 (1987) **[2]**; F. Béguin and R. Setton New ternary lamellar compounds of graphite, Carbon 13, 293-295 (1975). **[5]**

Advantageously, the polyaromatic compound may be naphthalene, benzophenone, fluorenone, benzoquinone or anthraquinone, preferably naphthalene. Advantageously, the polyaryl alkali salt may be a polyaryl potassium salt (i.e., a salt of the formula $M_0^+B^-$, wherein $M_0^+$ represents $K^+$). Advantageously, the polyaryl alkali salt of the formula $M_0^+B^-$ may be a naphthalene potassium salt ($Naph^-K^+$).

The reduction of the nanographite material may also be carried out by electrochemistry. The electrochemical reduction of the nanographite material takes place with the insertion of a counter cation present in the solution. Hodge, S. A., Fogden, S., Howard, C. A., Skipper, N. T. & Shaffer, M. S. P. Electrochemical Processing of Discrete Single-Walled Carbon Nanotube Anions. ACS Nano 7, 1769-1778 (2013). **[6]**

Advantageously, when the nanographite intercalation compound of step a) of the method of the present invention is prepared by reduction of a nanographite material in the presence of an alkali metal salt of formula $M_0^+B^-$, as described above, a filtration step can be performed after the formation of nanographite intercalation compound and prior to dissolving it in the aprotic organic solvent in step a). For example, when the preparation of nanographite intercalation compound involves a reduction in the presence of an alkali metal salt obtained from an alkali metal (for example, in the presence of a polyaryl alkali salt having the formula $M_0^+B^-$, as detailed above, filtration can allow separating the liquid phase (for example a solution of $K^+Napht^-$ in THF) from the solid phase comprising the nanocarbon intercalation compound. The resulting nanographite intercalated with an alkali metal can be rinsed one or more times with an appropriate solvent. The nanographite intercalation compound so rinsed may then be dried prior to dissolution in the aprotic organic solvent (A) or the mixture (A') of aprotic organic solvents.

The metal salt or metal complex used in step (b) may be derived from any suitable metal known in the art. For example, the metal may be selected from lanthanides, actinides, transition metals, and/or post-transition metals. Advantageously, the metal may be a transition metal selected from Fe, Ni, Pt, Rh, Mn, Cu and/or Co.

The metal salt or metal complex may be for example of formula $M(Z)_n$ wherein M represents the metal, each occurrence of Z independently represents a counterion or moiety to which the metal is complexed, and n the number of Z moieties depending on the valency of the metal M. In the formula $M(Z)_n$, the n occurrences of ligand Z may be the same or different. For example each occurrence of Z may independently be selected from $Cl^-$, Br, $I^-$, $OTF^-$, $BF^{4-}$, $PF^{6-}$, acetate, acetylacetonate or [(bis(trifluoromethylsulfonyl)amide)]. In other words, the metal salt or complex may be any suitable $Cl^-$, Br, $I^-$, $OTF^-$, $BF^{4-}$, $PF^{6-}$, acetate-, acetylacetonate- or [(bis(trifluoromethylsulfonyl)amide)]-based metal salts or complexes.

According to the invention, a suitable amount of at least one metal salt and/or at least one metal complex or a mixture of at least two metal salts and/or metal complexes may be an amount allowing to obtain a suspension of nanographene-supported metal nanoparticles. For example, the amount of at least one metal salt and/or at least one metal complex or a mixture of at least two metal salts and/or metal complexes may be adapted with regards to the composition of the nanographenide solution, notably its concentration in nanographenide species and/or the negative charge density on the surface of the nanographenide. For example, it may be up to equimolar in terms of the amount of metal salts/complexes vs the amount of negative charge accessible on the surface of the nanographenide.

When a single metal salt or metal complex $M(Z)_n$ is used in step b), wherein M, n and Z are as defined above, the process leads to the formation of nanoparticles of a single metal M anchored on nanographene; or of nanoparticles of a single metal oxide when the process comprises oxidation step c).

Alternatively, a mixture of two different metal salts or two different metal complexes, $M_1(Z_1)_{n1}$ and $M_2(Z_2)_{n2}$

may be used in step b) where $M_1$ and $M_2$ represent two different metals that will generate an alloy after reduction of the metal salts or metal complexes; $Z_1$ and $Z_2$, for each occurrence, independently represent a counterion or moiety to which the metal $M_1$ or $M_2$, respectively, is complexed; and n1 and n2 independently represent the number of $Z_1$ and $Z_2$ moieties, respectively, depending on the valency of the metal $M_1$ and $M_2$. In the formula $M_1(Z_1)_{n1}$, the n1 occurrences of ligand $Z_1$ may be the same or different. Likewise, in the formula $M_2(Z_2)_{n2}$, the n2 occurrences of ligand $Z_2$ may be the same or different. When $M_1$ and $M_2$ are suitably selected (i.e. metals that will generate an alloy after reduction of the metal salts or complexes), step b) of the process may lead to the formation of metal alloy nanoparticles anchored on nanographene. As such, when a mixture of metal salts or metal complexes is used in step b), the mixture may combine any two suitable metal precursors $M_1(Z_1)_{n1}$ and $M_2(Z_2)_{n2}$ (in the form of metal salts or metal complexes) where $M_1$ and $M_2$ may be any suitable metal couple that generates alloys known from one skilled in the art. The respective amounts of $M_1(Z_1)_{n1}$ and $M_2(Z_2)_{n2}$ used may be advantageously adjusted to fit the respective atomic ratio $M_1$:$M_2$ in the resulting alloy. Advantageously, the metals $M_1$ and $M_2$ may be selected from the following couples: Fe/Pt, Fe/Rh, Co/Pt and Co/Rh to generate the respective alloys. As such, step b) may comprise reacting the organic nanographenide solution obtained in step a) with a suitable amount of a Fe or Co salt or complex, and a suitable amount of a Pt or Rh salt or complex. The Fe, Co, Pt and Rh salts or complexes may be advantageously selected from Cl, Br, I, OTF, $BF_4$, $PF_6$, acetate-, acetylacetonate- or [(bis(trifluoromethylsulfonyl)amide)] salts or complexes of these metals. The alloy may be binary, ternary, quaternary or above, depending on the number of different metal salts or two different metal complexes used in step b). In step c), the metal alloy nanoparticles formed on the nanographene support may be oxidized, as described herein for metal nanoparticles. One metal component of the alloy may be preferentially oxidized. Alternatively, all metal components in the alloy may be oxidized.

In yet another variant, a mixture of at least two different metal salts or metal complexes, $M_1(Z_1)_{n1}$ and $M_2(Z_2)_{n2}$, may be used in step b) where $M_1$ and $M_2$ represent two different metals that will not generate an alloy; $Z_1$ and $Z_2$, for each occurrence, independently represent a counterion or moiety to which the metal $M_1$ or $M_2$, respectively, is complexed; and n1 and n2 independently represent the number of $Z_1$ and $Z_2$ moieties, respectively, depending on the valency of the metal $M_1$ and $M_2$.. In this case, the process may lead to the formation of at least two populations of nanoparticles anchored on nanographene: nanoparticles of metal $M_1$ and nanoparticles of metal $M_2$ (or metal oxide versions thereof when the process comprises oxidation step c). Nanoparticles of mixed metal $M_1$/$M_2$ may also be formed; i.e. nanoparticles of a mixture of metals $M_1$ and $M_2$ (without the structure of an alloy); or nanoparticles of mixed metal oxide versions thereof when the process comprises oxidation step c). As detailed above, in the formula $M_1(Z_1)_{n1}$, the n1 occurrences of $Z_1$ may be the same or different. Likewise, in the formula $M_2(Z_2)_{n2}$, the n2 occurrences of $Z_2$ may be the same or different. For example, each occurrence of $Z_1$ and $Z_2$ may independently be selected from $Cl^-$, Br, $I^-$, $OTF^-$, $BF^{4-}$, $PF^{6-}$, acetate, acetylacetonate or [(bis(trifluoromethylsulfonyl)amide)]. In other words, the metal salts or complexes, $M_1(Z_1)_{n1}$ and $M_2(Z_2)_{n2}$, may be any suitable $Cl^-$, Br, $I^-$, $OTF^-$, $BF^{4-}$, $PF^{6-}$, acetate-, acetylacetonate- or [(bis(trifluoromethylsulfonyl)amide)]-based metal salts or complexes. In this variant, $M_1$ and $M_2$, may take the definition of M, as defined above, namely, they may be, independently, any suitable metal known in the art. For example, $M_1$ and $M_2$ may independently be selected from lanthanides, actinides, transition metals, and/or post-transition metals. Advantageously, $M_1$ and $M_2$ may independently may be a transition metal selected from Fe, Ni, Pt, Rh, Mn, Cu and/or Co.

According to the invention, step a) of the method of the invention may be carried out with at least one metal salt and/or at least one metal complex or a mixture of at least two metal salts and/or metal complexes selected from:

(i) Fe, Ni, Co, Cu, and/or Mn salts of Cl, Br, I, OTF, $BF_4$, $PF_6$, acetate-, acetylacetonate- or [(bis(trifluoromethylsulfonyl)amide)] ; or

(ii) a mixture of:

- at least one Fe and/or Co salt of Cl, Br, I, OTF, $BF_4$, $PF_6$, acetate-, acetylacetonate- or [(bis(trifluoromethylsulfonyl)amide)]; and
- at least one Pt and/or Rh salt of Cl, Br, I, OTF, $BF_4$, $PF_6$, acetate-, acetylacetonate- or [(bis(trifluoromethylsulfonyl)amide)], for example $PtCl_2$ or $RhCl_3$.

According to the invention step b) may be carried out by addition, preferably dropwise addition, of a solution of the at least one metal salt and/or at least one metal complex or a mixture of at least two metal salts and/or metal complexes in an aprotic organic solvent, preferably solvent (A) or mixture (A') used in step a), to the organic nanographenide solution obtained in step a).

Without wishing to be bound by any particular theory, the addition of one metal salt and/or at least one metal complex or a mixture of at least two metal salts and/or metal complexes at step b) appears to trigger an electron transfer from the nanographenide to the dissolved metal cation, which leads to the formation of metal nanoparticles onto the

carbon surface. The reaction proceeds as long as the electron potential favors the metal particle generation.

According to the present invention, the oxidizing step c) which corresponds to the oxidation of metal nanoparticles present on the nanographene support may be carried out by exposing the suspension obtained in step b) to air and/or water and/or another oxidation agent. For example, when the suspension obtained in step b) is exposed to air and/or water in oxidizing step c), these conditions spontaneously provoke the oxidation of the metal nanoparticles present on the nanographene support.

The aforementioned oxidation agent may be any suitable oxidation agent that can provoke the oxidation of the metal nanoparticles present on the nanographene support, when placed into contact with the nanographene-supported metal nanoparticles obtained in step b), in place of air or water. The oxidation agent may be in liquid or gaseous form. For example it may be a gaseous mixture comprising ozone or iodine, or a mixture of $O_2$ and an inert gas. Alternatively, it may be a liquid solution comprising $H_2O_2$.

According to the invention, the exposition of the suspension obtained in step b) to air may be carried out through any suitable system known from the person skilled in the art. It may be for example by bubbling air through the suspension of nanographene-supported metal nanoparticles obtained in step b).

According to the invention, the exposition of the suspension obtained in step b) to water may be carried out by any method and/or process known from one skilled person. It may be, for example, by adding dropwise water in the suspension of nanographene-supported metal nanoparticles.

According to the invention, the exposition of the suspension obtained in step b) to an oxidation agent may be carried out by any method and/or process known from one skilled person. It may be, for example, by dropwise addition of an equimolar amount or slight excess of oxidation agent in the suspension of nanographene-supported metal nanoparticles.

According to the invention, the nanographene support of the nanographene-supported metal and/or metal oxide nanoparticle composite of the invention, may be single-layered nanographene, few-layered nanographene or a mixture of both.

Advantageously, the organic nanographenide solutions used in the context of the present invention would exhibit a finite number of electrons which advantageously according to the invention would allow tailoring the formation of nanoparticules of metal and/or metal oxide and/or metal alloy and/or metal alloy oxide. Advantageously, the present invention allows to prepare and obtain nanographene-supported metal and/or metal oxide nanoparticle composites, the metal and/or metal oxide nanoparticles having advantageously an average particle size in the lower nanometer range. For example, the metal or metal oxide nanoparticles present on the nanographene support may have a diameter from 0.5 to 100 nm, preferably from 0.5 to 20 nm, preferably from 0.5 to 15 nm, preferably from 0.5 to 10 nm, preferably from 0.5 to 5 nm, preferably from 1 to 5 nm, preferably from 2 to 5 nm.

According to the invention, the method may further comprise a step of anchoring one or more biocompatible groups on the carbon framework of the composite's nanographene support, or on the metal or metal oxide itself, either covalently or non-covalently.

According to the invention, the method for anchoring biocompatible groups on the carbon framework of the composite's nanographene support may be any method known from one skilled in the art suitable for the attachment of biocompatible groups on a carbon surface.

For example, the biocompatible groups may be anchored to the carbon framework by <u>non covalent</u> binding, for example via hydrogen bonds, van der Waals interactions, hydrophobic bonds, ionic bonds. For example, according to the polarity of the carbon framework, biocompatible groups having the opposite polarity may be anchored. For example, according to the lipophilic properties of the carbon surface, biocompatible groups having the same lipophilic properties which may form hydrophobic bonds.

According to the invention, the step of anchoring one or more biocompatible groups on the carbon framework of the composite's nanographene support may be carried out by <u>covalent</u> functionalisation of the carbon framework. It may be for example any method known from one skilled in art adapted to functionalize a surface. For example it may be any method known from one skilled in the art allowing to fix biomolecule on a surface. It may be for example a method as disclosed in Haeshin Lee et al. Facile Conjugation of Biomolecules onto Surfaces via Mussel Adhesive Protein Inspired Coatings Adv Mater. 2009 Jan 26; 21(4): 431-434. **[7]**

According to the invention, the step of anchoring one or more biocompatible groups on the composite's surface may be carried out by covalent or non covalent functionalisation of the metal and/or metal oxide and/or metal alloy and/or metal alloy oxide attached to the carbon framework of the composite's nanographene support. It may be for example any method known from one skilled in art adapted to functionalize a metal and/or metal oxide and/or metal alloy and/or metal alloy oxide. One skilled in the art taking into consideration its technical knowledge would know how to adapt the known methods to fix anchor biocompatible groups on surface.

As used herein, the expression "biocompatible group" means any suitable biocompatible group known from one skilled in the art. It may be for example a biocompatible group selected from the group comprising phosphonate ($PO_3^-$), sulfonate ($SO_3^-$), carboxylate ($COO^-$), hydroxyl ($OH^-$), albumin binding moieties, and phospholipid moieties.

The present invention also provides a nanographene-supported metal and/or metal oxide nanoparticle composite comprising metal and/or metal oxide nanoparticles grafted on nanographene. Advantageously, the nanographene support may be mostly (if not only) single-layered nanographene.

The nanographene, metal and/or metal oxide are as defined above in any variant.

In particular, the present invention provides a nanographene-supported metal and/or metal oxide nanoparticle composite obtainable by a method according to the present invention.

The present invention also provides a nanographene-supported Fe, Ni and/or Co oxide nanoparticle composite comprising iron oxide, nickel oxide and/or cobalt oxide nanoparticles grafted on nanographene; wherein the composite exhibits ferromagnetic properties. Advantageously, the nanographene support may be mostly (if not only) single-layered nanographene.

The present invention also provides a nanographene-supported Fe/Pt, Fe/Rh, Co/Pt and/or Co/Rh metal alloy nanoparticle composite comprising Fe/Pt, Fe/Rh, Co/Pt and/or Co/Rh metal alloy nanoparticles grafted on nanographene; wherein the composite exhibits magnetic properties. Advantageously, the nanographene support may be mostly (if not only) single-layered nanographene.

The present invention also provides a nanographene-supported Fe/Pt, Fe/Rh, Co/Pt and/or Co/Rh metal alloy oxide nanoparticle composite comprising Fe/Pt, Fe/Rh, Co/Pt and/or Co/Rh metal alloy oxide nanoparticles grafted on nanographene; wherein the composite exhibits magnetic properties. Advantageously, the nanographene support may be mostly (if not only) single-layered nanographene.

According to the present invention, the metal oxide or metal alloy nanoparticles grafted on nanographene may have a nanomolecular size. For example, the metal oxide or metal alloy particles grafted on nanographene may have a particle size (i.e., diameter) from 0.5 to 100 nm, preferably from 0.5 to 20 nm, preferably from 0.5 to 15 nm, preferably from 0.5 to 10 nm, preferably from 0.5 to 5 nm, preferably from 1 to 5 nm, preferably from 2 to 5 nm.

Advantageously, the nanographene support may be turbostratic few layer nanographene of average lateral size 10-100 nm, preferably 10-50 nm, most preferably 10-30 nm. Such turbostratic few layer nanographene may originate from nanographite obtained from microwave plasma splitting of methane/carbon dioxide mixture.

According to the invention, the nanographene-supported metal and/or metal oxide nanoparticle composite may have one or more biocompatible groups anchored on the carbon framework of the composite's nanographene support, or on the metal or metal oxide nanoparticle itself, either covalently or non-covalently.

Advantageously, one or more biocompatible groups may be anchored, to the surface of the metal oxide or metal alloy nanoparticles grafted on the nanographene support.

The inventors have demonstrated that the nanographene-supported metal and/or metal oxide nanoparticle composite of the present invention have advantageous properties, in particular due to the structure of the nanographene support and also the size of the metal or metal oxide nanoparticles grafted on said support.

In particular, the inventors have surprisingly demonstrated that the obtained composites, for example comprising Fe particles, are as efficient for fuel cell technology as platinum based electroctalysts without the problem of cost and life cycle of platinum.

Accordingly, the present invention also refers to use of a composite of the invention as catalytic material.

The inventors have also surprisingly demonstrated that the obtained composites, particularly those containing Fe, Co or Ni oxide nanoparticles or Fe or Co alloy nanoparticles, have advantageously magnetic properties of these materials which could allow magnetic separation when applied as a catalysts for chemical reactions. In particular, the inventors have demonstrated that the composite of the invention, for example comprising iron oxide nanoparticles, surprisingly exhibit magnetic properties with room-temperature, i.e. 20 to 25°C, magnetic remanence and magnetic hysteresis. These magnetic properties of the composites of the invention are really surprising since iron oxide particles alone (i.e., no nanographene support) in the range of 2-5 nm do not show any magnetic properties in the same conditions.

The inventors have also demonstrated that the composites of the invention are advantageous catalysts with high surface area, potentially as efficient as homogeneous catalysts without the separation problems associated with those. The inventors have also demonstrated that the composites of the invention are advantageously, easily separable from a reaction mixture as heterogeneous catalysts without the low activity associated with those (pores, reagent and product diffusion, and so on...). This separation can be performed magnetically, by sedimentation, filtration or centrifugation.

The inventors have also surprisingly demonstrated that the obtained composites are efficient BIFUNCTIONAL electrocatalysts, i.e. they work for OER (fuel cells) but also for ORR (energy storage).

In addition, the inventors have demonstrated that the composites of the invention provide more effective electrocatalytic sites, i.e. the number of metal and/or metal oxide nanoparticles, present on the composites, which can be electrochemically oxidized and reduced is 3 to 30 times higher than known metal electrocatalysts in other form, for example porous Platinum or solid Platinum.

Accordingly, the present invention also relates to use of a composite of the invention as electrode material for use in electrocatalysis.

According to the invention, the composite of the invention may be used in electrocatalysis for applications in fuel cell and energy storage technology. For example, the composite of the invention may be used as electrocatalyst for oxygen reduction reaction (ORR) or oxygen evolution reaction (OER).

Advantageously, since the composite according to the invention is an efficient bifunctional electrocatalyst for oxygen reduction reaction (ORR) and oxygen evolution reaction (OER), the composite of the invention may be used as bifunctional electrocatalyst for oxygen reduction reaction (ORR) and oxygen evolution reaction (OER).

According to the invention, the composite of the invention may be used as catalyst for gas-phase chemical reactions. According to the invention, the gas-phase chemical reactions may be any gas-phase chemical reactions known from one skilled in the art in which a catalyst is required or helpful. For example it may be hydrogenation of CO in the Fischer-Tropsch process.

According to the invention, the composite of the invention may be used as slurry catalyst for liquid-phase chemical reactions. According to the invention, the liquid-phase chemical reactions may be any liquid-phase chemical reactions known from one skilled in the art in which a catalyst is required or helpful. For example it may be liquid phase oxidation processes, for example oxidations of aldehydes to carboxylic acids, benzyl alcohol to benzaldehyde, vanillyl alcohol to vanillin, or liquid phase reduction reactions.

Advantageously, when the composite of the invention is used as slurry catalyst for liquid-phase chemical reactions, and when the metal oxide is a Fe, Ni or Co oxide or a mixture of at least two of them, the composite may be separated from the reaction mixture by application of a magnetic field.

According to the invention, the composite of the invention may be used for water purification and waste water treatment. According to the present invention, water purification and waste water treatment may be any water purification and waste water treatment known from one skilled in the art involving catalytic reactions and/or using activated carbon filter. Advantageously, the composites of the invention allow to improve the efficiency of the water purification and waste water treatment. Advantageously, the composite of the present invention may be used in water purification and waste water treatment for removing toxic metals, such as arsenic, from ground water.

According to the invention, when the composite comprises one or more biocompatible groups anchored on the carbon framework of the composite's nanographene support, either covalently or non-covalently, or on the metal itself, it may be advantageously used as a component of a drug or be used as medicament.

The inventors have also demonstrated that the composite of the invention have magnetic properties which could be useful for bio medical technology, in particular as further molecules may be adsorbed on the carbon surface, dragged to the desired position and been released there over time. Hyperthermia also benefits from composite of the invention showing open magnetic hysteresis close to room temperature.

According to the invention, the composite may be used as a drug in the treatment of cancerous tumors by hyperthermia.

Other advantages

The inventors have demonstrated that the present invention allows successful intercalation of nanographite material, dissolution in an aprotic organic solvent and the use of the resulting nanographenide solution as efficient reducing agent for the preparation of composite materials consisting of nano-sized graphene sheets decorated with metal and/or metal oxide nanoparticles. In other words, the present invention allows to effectively produce nanographene-supported metal and/or metal oxide nanoparticle composites.

The inventors have also demonstrated that the organic nanographenide solution in the process of the invention, are advantageous compared to known processes in that:

i) since nanographenide solutions are strong reducing agents by themselves, optionally avoiding addition of any additional reducing agent,

ii) the redox reaction takes place in close proximity to the carbon lattice, as the electrons are provided by the nanographite intercalation compound and the decoration of the carbon framework will thus proceed efficiently

iii) the amount of reduction agent can be controlled by the concentration of the respective nanographenide solution used,

iv) there is no need to any stabilization or dispersing agents that could interfere with the formation mechanism

v) the method of the invention allows to generate a broad variety of possible different metal/metal oxide nanoparticles that can be attached on the carbon framework

vi) very small nanoparticles of metal and/or metal oxide adhere to the nanographene flakes, preventing leaching of material and allowing a high catalytic activity.

Brief description of the Figures

Figure 1 is a schematic representation for the synthesis of the composite material Fe(np)/nC containing iron oxide nanoparticles with defined sizes attached to the carbon lattice. A) intercalation of the nanographite starting material (nC) with potassium metal yielding $KC_8$, B) dissolution of the $KC_8$ in absolute Tetrahydrofuran (THF) by the aid of stirring and centrifuging the sample afterwards to obtain the nanographenide solution, C) reaction of nanographenide solutions with dissolved ($Fe(BF_4)_2$), in absolute THF, followed by aqueous work up and isolation of Fe(np)/nC.

Figure 2 are photographs of a) High-resolution transmission electron microscopy (HR-TEM) images of smaller aggregates formed in the composite material Fe(np)/nC b) higher magnification on the composite material, revealing the atomic planes of the particles indicated by arrows. c) scanning tunneling microscope (STM) images of the composite material deposited on $MoS_2$. Fe nanoparticles are indicated by arrows.

Figure 3 is a graphic representing the XPS survey scan in the region between 50-780 eV of the composite material Fe(np)/nC in comparison to the nanographite (nC), peaks for iron, oxygen and carbon are found as indicated. The Y-axis represents the counts (a.u.) and the X-axis the binding energy in electron Volt (eV).

Figure 4 are graphics representing the electrochemical characterization of Fe(np)/nC electrodes. The Y-axis represents $J/mAcm^{-2}$ and the X-axis E-iR/V. a) Comparison of ORR onset potentials for bare GC electrode, the pristine nanographites (nC) and the Fe(np)/nC catalyst. CVs at 10 mV/s in KOH 0.1 M, rotation speed 1600 rpm. The Fe(np)/nC line corresponds to the Fe(np)/nC catalysts in absence of oxygen. b) RDE experiments at different rotation speeds (from top to bottom: 400, 900, 1600, 2500 and 3600 rpm) for Fe(np)/nC. Catalyst loading: 140 $\mu g/cm^2$. Inset: respective Koutecky-Levich analysis (current of the positive-going CV scans taken at +0.55 VRHE as a function of the rotation speed) that highlights the high number (four) of transferred electrons.

Figure 5 are graphics representing a) Comparison of OER performances for different Fe(np)/nC electrodes of different loading: 70, 140 and 350 $\mu g/cm^2$. CVs at 10 mV/s in $O_2$-saturated ultrapure 0.1 M KOH, without stirring the solution. b) Potentiostatic electrolysis at E = 1.63 VRHE ($\eta$ = 0.4 V) for a representative Fe(np)/nC electrode having a catalyst loading of 140 $\mu g/cm^2$. Inset: quantification of electroactive Fe species by coulometry of the FeO peak for a Fe(np)/nC electrode before and after electrolysis. The Y-axis represents $J/mAcm^{-2}$ and the X-axis time in seconds.

Figure 6 are graphics representing a) Tafel plots for a Fe(np)/nC catalyst (140 $\mu g/cm^2$) obtained from CVs at 10 mV/s. Solid lines refer to ORR experiments, dashed line to OER experiments. The Y-axis represents Overpotential/V and the X-axis $J/mAcm^{-2}$. b) Comparison of the combined overpotential for ORR and OER for Fe(np)/nCs and recently reported bifunctional catalysts for oxygen electrocatalysis. $\eta$OER are referred to a current density of 10 $mA/cm^2$, while $\eta$ORR @ -1 $mA/cm^2$.

Figure 7 are respectively AFM image (a) and graphics (b, c, d), b is cross section along the white line of AFM image, the Y-axis represents the height (nm) and the X-axis lateral dimension (nm), c) statistical analysis of the lateral size, the Y-axis represents the frequency (a.u) and the X-axis lateral size (nm), d) data of the height distribution, Y-axis represents the frequency (a.u) and X-axis height (nm).

Figure 8 a) and b) are bright field TEM image and corresponding STEM/EDX element mapping of the iron K edge of a bigger aggregate.

Figure 9 are TEM images of the three different composite material, a) Fe(np)/nC($Fe(BF_4)_2$), b) Fe(np)/nC($FeCl_3$), c) Fe(np)/nC($FeCl_2$). The composite materials aggregate and exhibit ensamble up to the $\mu$m length scale, mainly composed by round-shaped objects randomly distributed.

Figure 10 are HR-TEM images of Fe(np)/nC($Fe(BF_4)_2$), scale bar of 2 nm. The focus was varied in this series, a) focused on iron nanoparticle b) -10 nm of a) c) +10 nm of a).d) HR-TEM images of Fe(np)/nC($Fe(BF_4)_2$) with a scale bar of 2 nm on another aggregate focused on the nano particle. e) +1 nm defocused to d), f) +2nm defefocused to d). The orientation of the carbon lattice is constant over the series of pictures whereas the crystal structure of the

grafted iron nanoparticles becomes visible in a) and vanishes again when out of focus (panel b,c).

Figure 11 are respectively AFM image of Fe(np)/nC deposits on freshly cleaved mica surface (a) and graphics (b, c, d), b is a cross section of the topography image taken along the white line are plotted in inserted figure, the Y-axis represents the height (nm) and the X-axis lateral dimension ($\mu$m), c) statistical analysis of the lateral size, the Y-axis represents the frequency (a.u) and the X-axis lateral size (nm), d) data of the height distribution, the Y-axis represents the frequency (a.u) and the X-axis height (nm).

Figure 12 are graphics representing a) XPS survey scan in the region between 50-780 eV of three composite materials Fe(np)/nC ($Fe(BF_4)_2$), Fe(np)/nC ($FeCl_3$) and Fe(np)/nC ($FeCl_2$) in comparison to the nanographite (nC) indicate O, Fe and C peaks in the figure. The Y-axis represents the counts (a.u) and the X-axis the binding energy (eV). b) high resolution XPS measurement in the iron region (700-740 eV) for three composite samples Fe(np)/nC ($Fe(BF_4)_2$), Fe(np)/nC ($FeCl_3$) and Fe(np)/nC ($FeCl_2$), which exhibit comparable features without regard of the iron salts used during the synthesis. The Y-axis represents the counts (a.u) and the X-axis the binding energy (eV).

Figure 13 is a graphic of XRD measurements of three different composite materials FeNP/nC (FeNP/nC($Fe(BF_4)_2$), FeNP/nC ($FeCl_3$) in and FeNP/nC ($FeCl_2$)) and the nC in comparison to reference spectra of $Fe_2O_3$ and FeOOH. The Y-axis represents the counts (a.u) and the X-axis $2\theta$ (degree).

Figure 14 is a graphic representing a comparison of ORR performances for bare GC substrate, the starting nC material and three Fe(np)/nC electrodes with different catalyst loading (70,140 and 350 $\mu g/cm^2$). CVs at 10 mV/s in $O_2$-saturated ultrapure (99.99%) 0.1 M KOH. Rotation speed: 1600 rpm. The Y-axis represents the current density and the X-axis the electrochemical potential.

Figure 15 represents different Koutecky-Levich plots for Fe(np)/nC electrodes with a catalyst loading of a) 70 $\mu g/cm^2$, b) 140 $\mu g/cm^2$ and c) 350 $\mu g/cm^2$. The analysis reveals comparable values for the number of transferred electrons with the exception of the lowest loading, where exposure of the underlying GC to the solution likely occurs.

Figure 16 represents the electrochemical measurements normalized for the amount of electroactive iron. CVs in a) the ORR and b) the OER regions normalized for the amount of electroactive Fe species as determined by CV. Scan rate: 10 mV/s, ultrapure KOH 0.1 M. The Y-axis represents the normalized current / a.u. and the X-axis the electrochemical potential.

Figure 17 represents TEM images of the composite material using (a,b) nanographite, and (c,d) large flake size natural graphite, respectively, as starting material for intercalation. The respective particles are about one order of magnitude smaller in the former case.

## EQUIVALENTS

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.

The following examples contain important additional information, exemplification and guidance that can be adapted to the practice of this invention in its various embodiments and the equivalents thereof.

## EXEMPLIFICATION

The method of this invention and its applications can be understood further by the examples that illustrate some of the embodiments by which the inventive method may be reduced to practice. It will be appreciated, however, that these examples do not limit the invention. Variations of the invention, now known or further developed, are considered to fall within the scope of the present invention as described herein and as hereinafter claimed.

The present invention will now be exemplified using certain metal and/or metal oxide materials but it will be understood this is not meant to limit the invention to those specific materials.

## EXAMPLES

### Example 1: Preparation of nanographene-supported metal and/or metal oxide nanoparticle composites

a) Purification of the nanographite starting material
The starting nanographite has been produced using microwave plasma technology and its characteristics have been previously reported (Ferdinand Hof, Katerina Kampioti, Kai Huang, Christèle Jaillet, Alain Derré, Philippe Poulin, Hisham Yusof, Thomas White, Krzysztof Koziol, Catharina Paukner, Alain Pénicaud, Conductive inks of graphitic nanoparticles from a sustainable carbon feedstock, Carbon 111 (2017), 142-149).
Purification of the raw nanographite was achieved by controlled heat treatment in a Nabertherm oven under air atmosphere. The solid raw nanographite was heated at a rate of 5°C / min until 500°C, and after a 6 h plateau cooled down to 30°C at 5°C / min and finally collected.
b) Synthesis of nanographite intercalation compound. 120 mg (10.0 mmol) of purified nanographite, and 48.8 mg (1.25 mmol) cleaned potassium (stoichiometry KC8) have been placed together in a vial inside an argon filled glove box and heated for 5 hours at 180°C on a heating plate under occasional stirring. Afterwards, the vial has been allowed to cool down to room temperature and the intercalated nanographite has been collected. The resulting salt has been directly used to prepare the following nanographenide solutions and also for the characterization of the compound (see example 2).
c) Dissolution of nanographite intercalation compound (KC8) and isolation of the nanographenide solution. 150 mg of $KC_8$ have been mixed with 150 mL of absolute Tetrahydrofuran (THF) under inert conditions, i.e. under argon atmosphere < 1 PPM oxygen/ water in a 250 mL Erlenmeyer flask and the mixture was stirred for 1 day, i.e. 24 hours, by the aid of a glass coated magnetic stirring bar. The resulting mixture was then centrifuged at 4500 rpm for 30 min under inert conditions and the faint yellowish upper solution was retained (nanographenide solution). The concentration of the nanographenide solution has been determined by dry extracts as 0.20 mg/mL $\pm$ 0.02 mg/mL. The nanographenide solution has been directly used for analysis and for nanoparticles synthesis.
d) Synthesis of the composite compounds. 100 mL of nanographenide solution obtained in a) was placed in a 250 mL Erlenmeyer flask inside an argon filled glove box. Afterwards, 11.2 mg (74 $\mu$mol, 0.5 eq.) of recrystallized iron tetrafluoroborate salt dissolved in 10 mL of absolute THF was added dropwise to the nanographenide solutions. Other Fe salts have also been used (see Table 1 below) yielding very similar products.

**Table 1**: Synthesis details regarding the preparation of Fe(np)/nC composite materials

| | Volume of nanographenide solution (mL) | Weight of solvated intercalation compound (mg) | Amount of electrons ($\mu$mol) | Amount of added iron metal salt ($\mu$mol) | Weight of added iron metal salt (mg) |
|---|---|---|---|---|---|
| Fe(np)/nC ($Fe(BF_4)_2$) | 100 | 20 | 148 | 74 | 11.2 |
| Fe(np)/nC ($FeCl_3$) | 100 | 20 | 148 | 50 | 8.3 |
| Fe(np)/nC ($FeCl_2$) | 100 | 20 | 148 | 74 | 9.3 |

After a brief period (5-15 min), aggregation and precipitation occurred. Subsequently, the dispersion was stirred for 24 hours and then removed from the glove box. The flask was exposed to ambient conditions and 100 mL of deionized water were added to the dispersion. The sample was isolated and purified by repeated centrifugation and redispersing steps (4 times, 1 h at 10 000 rpm). The final composite materials Fe(np)/nC was finally isolated by freeze drying.
In summary, the composite material Fe(np)/nC has been prepared by (i) synthesis of the intercalation compound from nanographite (nC), (ii) isolation of the nanographenide solution after stirring and centrifugation of $KC_8$ in THF, (iii) reaction of the nanographenide solution with ($Fe(BF_4)_2$) salt (Figure 1, A, B and respectively). All reactions, with exception of the final work up, have been carried out in a glove box under argon atmosphere. During the last purification steps, the prepared metallic iron nanoparticles get oxidized, forming the final composite materials Fe(np)/nC under study. Beside the ($Fe(BF_4)_2$) salts, $FeCl_3$ and $FeCl_2$ salts have also been exploited in the synthesis. The respective products behave very similarly.
e) Characterization of the nanographene-supported metal and/or metal oxide nanoparticle composites
Raman analysis of the nanographite starting material (**nC**) shows typical D, G, D' and 2D graphitic Raman bands. It is noteworthy that the D-band in this specific nC originates quasi exclusively from edge contribution. In sharp

contrast, the intercalated **nC** with stoichiometry $KC_8$ exhibits a spectral profile with complete absence of these bands. Instead, the typical profile of graphitic $KC_8$ GIC with a broad featureless signal around 1500 is observed.

Nanographenide solutions were obtained upon stirring. Size analysis of the nanographenide solution by atomic force microscopy (AFM) reveals that the sample has a homogeneous size distribution, with a lateral size of about 15-30 nm and a height of -0.5-1.5 nm.

In the composite material Fe(np)/nC prepared by using $Fe(BF_4)_2$ as metal salt, the presence of both, iron and carbon in the obtained composite material Fe(np)/nC was confirmed by TGA measurements under air. Comparative TGA measurement of the starting nanographite (nC) reveals that complete combustion of the carbon lattice occurs at about 650-750°C under air and no remaining mass is observable. In stark contrast, the TGA profile of the Fe(np)/nC exhibits a remaining mass of about 24 %. Table 2 below discloses the iron content of different composite material after TGA measument.

**Table 2**: Determination of the iron content in the samples after TGA measurement under air between 30-750°C

| Sample | Decomposition temperature (°C) | Total amount of sample measured (mg) | Remaining mass (mg) | Amount of $Fe_2O_3$ ($\mu$mol) | Fe content (at %) |
|---|---|---|---|---|---|
| nC | 650 | 4.31 | 0.0 | 0.0 | 0.0 |
| Fe(np)/nC ($Fe(BF_4)_2$) | 400 | 5.65 | 1.33 | 8.3 | 2.3 |
| Fe(np)/nC ($FeCl_3$) | 415 | 4.27 | 0.97 | 6.0 | 2.2 |
| Fe(np)/nC ($FeCl_2$) | 415 | 5.04 | 1.36 | 8.5 | 2.7 |

As shown in Table 2 above showing the Thermogravimetric analysis (TGA) measurements under air between 30-750°C of three different composite materials Fe(np)/nC (Fe(BF4)2), Fe(np)/nC (FeCl3) and Fe(np)/nC (FeCl2) versus the nC, the combustion of the carbon lattice occurs between 400-415°C. Decomposition of the carbon lattice was observed for the starting nC at about 650°C, whereas the decomposition of the carbon lattice for the three composite materials was between 400-450°C, the main decomposition temperature has been determined between 426 -438 °C. A comparison with the starting nC highlights a shift of more than 200°C towards lower degradation temperature, exemplifying the catalytic activity of the Fe nanoparticles on the carbon combustion.

The composite materials were further studied by means of high resolution electron transmission microscopy (HR-TEM), scanning tunnel microscopy (STM) and by scanning TEM in combination with energy dispersive x-ray (EDX) analysis. Representative images the composite materials are shown in Figure 2a, 2b and Figures 8-10, respectively. In the respective images the size and shape of the carbon lattice, as well as the crystalline domains of the iron oxide species, are seen. Aggregates of the composite materials are predominantly present as clearly shown on Figure 9. Those exhibit a broad distribution of different sizes, ranging from about 50 nm, up to several $\mu$m, and have a round-shaped appearance. HR-TEM images of the smaller aggregates reveal that this appearance corresponds to graphene flakes randomly oriented (Figure 2b). The presence of small crystalline domains randomly distributed on the carbon lattice was seen only in a higher magnification. These nanoparticles are located both in between the carbon flakes and on top of the carbon lattice and have sizes of 2-5 nm. (Figure 10). The bright field image and corresponding EELS measurement of the sample show the presence of the iron particles as well as the carbon flakes. It is apparent that the iron nanoparticles are found only on the carbon framework, both by electron energy loss spectroscopy (EELS) and STEM/EDX analysis.

Figure 2c is a representative STM image showing individual nanographene flakes. In this image, the surface (MoS2) with aggregates of -50 nm and nanoparticles, exhibiting a different conductivity pattern, can clearly be seen. The measured objects appear not to be lying flat on the surface but to be crumpled, when comparing these STM images to known prepared from aqueous dispersions. The presence of the nanocomposite was further confirmed by AFM (Figure 11).

In addition, a chemical analysis of the nanographene-supported metal and/or metal oxide nanoparticle composites was carried out. The presence of the iron and its quantification in the composite materials was further corroborated by XPS measurements. The obtained results are illustrated on Figure 3. In the survey scans (Figure 3) only carbon and a minor content of oxygen was observed for the starting nC and for the composite the elements carbon, iron and oxygen were detectable. In the composite sample several peaks can be found in the high resolution spectra in the iron region between 700-740 eV (Figure 12). By fitting the respective data, the amount of iron in the samples and the chemical nature of the iron species were determined. Based on such XPS measurements, values between 2.5 at % and 3 at %

iron were calculated and are presented in Table 3 below.

**Table 3:** Calculation of the carbon, oxygen and iron content generated by fitting the respective XPS data

| Peak | Position (ev) | FeNP@NC ($Fe(BF_4)_2$) At % | FeNP@NC ($FeCl_3$) At % | FeNP@NC ($FeCl_2$) At % |
|---|---|---|---|---|
| $C_{1s}$ | 284.56 | 81.65 | 80.6 | 83.1 |
| $O_{1s}$ | 530.11 | 5.65 | 7.02 | 5.23 |
| $O_{1s}$ | 531.01 | 3.03 | 3.29 | 6.95 |
| $O_{1s}$ | 531.76 | 4.70 | 3.26 | 0.58 |
| $Fe_{2p3/2}$ | 710.90 | **2.61** | **2.98** | **2.49** |

The respective fits show major peaks at about 711 eV and 714 eV, which can be assigned to iron(III) oxide and iron(III) hydroxide species. Further information about the chemical nature of the oxygen was retrieved from measurements in the region between 520-540 eV. In the pristine starting material, a peak at about 532 eV corresponding to CO binding motifs was detected, whereas in the three composite materials, two to three new peaks were present between 530.0 eV and 531.5 eV.

By means of detailed XRD studies illustrated with Figure 13, the presence and the chemical structure of the iron oxide species in the composite material was further investigated. In the spectra of the starting nC, peaks at 25.6° and 42.3° correspond to the ordered graphitic carbon lattice, whereas for the composite materials several new peaks were found. These peaks can be found about 33, 36, 49 and 53° and are very broad. Statistical Raman histograms for the composite materials Fe(np)/nCs were elaborated, which demonstrated an increase in the A(D/G). Analysis of the respective average gamma G vs A(D/G) showed that the mean value is shifted towards higher A(D/G) values. This clearly demonstrates that the composite materials Fe(np)/nCs have a much lower content of functionalization with oxygen or hydrogen species at the carbon basal plane.

In the nanographenide solution after centrifugation, reduced carbon sheets are present exhibiting 1-4 layers of nanographene and sizes of about 15-25 nm (Figure 7). By addition of different iron salts to the nanographenide solution, an electron transfer from the nanographene sheet was triggered, leading to the formation of iron nanoparticles and potassium salts. The potassium tetrafluoroborate salt is easily removed by aqueous work up, providing composite materials composed solely of carbon, iron and oxygen.

XPS measurements corroborate the observation that the final Fe(np)/nC material is composed only by the elements iron, carbon and oxygen (Figure 3). The presence of predominantly iron(III) in the samples is confirmed by peaks (about 711 eV and about 714 eV) and 2-3 peaks for oxygen as well as hydroxide bound to iron(III) (530-531 eV), which are not present in the pristine material, being in a good agreement to the XRD data. From the respective fits in the iron region, the amount of iron in the samples has been estimated to have values in the range of 2.5 to 3.0 at%. These values are furthermore validated and corroborated by means of TGA measurements under air (see Table 2) and by EELS measurement. The combustion temperature is shifted to lower temperatures for about 650-750°C for the pristine material to about 400°C in the composite material. The observed effect is related to the intrinsic catalytic behavior of the attached nanoparticles that triggers the decomposition of the carbon lattice at lower temperature. HR-TEM measurements reveal that the iron particle have a size about 2-5 nm (Figures 2, 9, 10) and are well distributed only on the carbon flakes (see also EELS and STEM/EDX results, Figure 8). The nanoparticles are distributed randomly on the carbon framework and do not exhibit any obvious ordering.

This example demonstrated the successful intercalation of nanographite, its dissolution in absolute THF and the exploitation of the derived nanographenide solution as efficient reducing agent for the preparation of composite materials consisting of nanographene sheets decorated with metal and/or metal oxide, for example iron oxide, nanoparticles (Fe(np)/nC).

This example clearly demonstrates that an example of process according to the invention allows to obtain a nanographene-supported metal and/or metal oxide nanoparticle composites. In addition, this example clearly demonstrates that the reaction is controllable, reliable and can be applied to a broad variety of different metal precursors, allowing the synthesis of varied electrocatalytic active composite materials.

**Example 2: determination of the electrochemical activity/properties of nanographene-supported metal and/or metal oxide nanoparticle composites**

The electrochemical properties of Fe(np)/nC obtained in Example 1 have been evaluated in a three-electrode electrochemical cell using a glassy carbon (GC) rotating disk electrode (RDE, Tacussel, France) as support for the

deposition of different catalyst inks, a Standard Calomel reference electrode (SCE) and a Pt mesh auxiliary electrode. The electrochemistry work stations used for the entire characterization was a SP-300 bipotentiostat (Biologic Instruments), having an additional current booster and a built-in Electrochemical Impedance Spectroscopy (EIS) analyser. The Fe(np)/nC powder was dispersed in THF (0.5 mg/mL) and dropcasted (5 μL) onto the RDE substrate, adjusting the deposited volume so as to prepare electrodes with different catalyst loading (70, 140 and 350 μg/cm$^2$). The bi-functional electroactivity of the Fe(np)/nC composite material for oxygen reduction reaction (ORR) and oxygen evolution reaction (OER) was evaluated separately by independent experiments in $O_2$-saturated 0.1 M ultrapure KOH (99.99%, Sigma-Aldrich).

The activity of Fe(np)/nC for ORR and OER has been addressed by comparing the catalyst composite material with the pristine nanographite material without the presence of Fe(np) and also with the supporting GC RDE electrode by itself (Figures 4 and 5). Oxygen reduction reaction: As evidenced in Figure 4a, a comparison of the CVs for the three systems reveals the catalytic effect provided by the Fe(np)s. The onset potential of ORR for Fe(np)/nC is at +0.8 VRHE, about 200 mV above that on a bare GC electrode and 150 mV above that with un-functionalized pristine NC material. For sake of comparison, the background CV obtained degassing the solution with argon does not show any faradaic process in the investigated potential window. A more detailed investigation of ORR revealed a dependence of the onset potential and, more importantly, of the limiting current on the catalyst loading. With the aim to reduce as much as possible the amount of catalysts on the electrodes, three different set of Fe(np)/nC electrodes at loadings of 70, 140 and 350 μg/cm$^2$ have been prepared. The 140 μg/cm$^2$ loading gives nearly as efficient limiting current density (J = -3.6 mA/cm$^2$) as the 350 μg/cm$^2$ loading (J = -4 mA/cm$^2$) but with a much better yield in term of electroactive Fe content (44 % against 20 %, see Figure 14 and Table 4).

Table 4 below presents the results of comparison between electrodes with different catalyst loading and the electrochemical data thereof obtained.

**Table 4:** Comparison between electrodes with different catalyst loading and the electrochemical data thereof obtained.

| Volume of Fe(np)/nC deposited | Loading of Fe(np)/nC (μg/cm$^2$) [a] | Loading of Fe (μg/cm$^2$) [b] | Charge of $Fe^{0/II}$ (mC) [c] | Maximum Charge of $Fe^{0/II}$ (mC) [d] | (Electroactive iron / Total iron) |
|---|---|---|---|---|---|
| 10 μL | 70 | 8 | 0.57 | 1.87 | **30%** |
| 20 μL | 140 | 16 | 1.65 | 3.74 | **44%** |
| 50 μL | 350 | 40 | 1.84 | 9.3 | **20%** |

[a] catalyst loading calculated considering a concentration of the catalyst in the ink of 0.5 mg/mL, [b] Fe loading calculated from the mean value of iron content in Fe(np)/nC of 2.6 at.% as evaluated by XPS measurements, [c] as determined by CV, [d] The maximum charge of $Fe^{0/II}$ oxidation is calculated again from the mean value of iron content in Fe(np)/nC (2.6 at.%) as evaluated by XPS measurements.

In order to understand the mechanism involved, the limiting current of ORR was then measured at different rotation speeds (Figure 4b) and the number of electrons (n) involved in the reaction is obtained from the slope of the so-called Koutecky-Levich (K-L) plots ($_{iRDE}$ vs. $\omega^{-1}$, where $\omega$ is the angular velocity, inset to Figure 4b).

$$n = \frac{1}{0.62bFAC_0D^{2/3}v^{-1/6}}$$ Eq. 1

where F is the Faraday constant, $C_0$ is the oxygen concentration in 0.1 M KOH (8.4 x $10^{-7}$ mol $cm^{-3}$), A is the electrode surface area (0.071 $cm^{2}$), D is the diffusion coefficient for $O_2$ in 0.1 KOH at RT (2 x $10^{-5}$ $cm^2$ $s^{-1}$) and ν the kinematic viscosity (0.008977 $cm^2$ $s^{-1}$).

All the Fe(np)/nC electrodes displayed very similar results in terms of exchanged electrons (n 4, measured at +0.55 VRHE), without a marked dependence on the catalyst loading (Figure 15). The high electron transfer numbers suggests high $H_2O$ yields, indicating the selective production of water rather than $H_2O_2$. Furthermore, the good reversibility observed for all scans on Figure 4b shows stability of the electrocatalysts vs polarization during the timescale of the experiment.

Oxygen evolution reaction: Furthermore, the uncommon activity of Fe(np)@NC in the context of oxygen electrocatalysis resides in its ability to also catalyze water oxidation (i.e. OER). As clearly evidenced in Figure 5a, no activity at all is observed for both the pristine nanographite (nC) and the GC substrate whereas Fe(np)/nC electrodes exhibit amazing catalytic activity for OER with onset overpotential at ca 1.55 VRHE.

The stability of Fe(np)/nC over time was demonstrated by potentiostatic electrolysis at ηOER = 0.4 V (Figure 5b). An hour long chronoamperometry for a representative electrode having a catalyst loading of 140 μg/$cm^2$ shows how after an initial increase of current the current signal remains very stable and constant throughout the duration of the experiment.

Fe activity: When comparing the activity of Fe(np)/nC to that of the metal-free starting nC, the catalytic role of the composite material, i.e. the role of the Fe(np)s, was observed. That in fact the Fe NPs being the active sites for both ORR and OER can be argued when considering the charge related to the reduction/oxidation of Fe(II)/Fe(0) species, i.e. the amount of electroactive iron. The charge associated with the typical redox processes of iron (FeII/0 reduction and Fe0/II oxidation) increases as the loading of Fe(np)/nC catalyst increases.

Further analysis of the same redox processes highlights also the stability of the investigated Fe(np)/nCs catalysts toward the potential dissolution of the Fe NPs under anodic polarization (inset of Figure 5b). A comparison of the CVs before and after one hour potentiostatic experiments at +1.63 VRHE reveals that the amount of electroactive Fe species (QFe) is constant, with no evidences of degradation of the catalyst in the investigated timeframe (QFe = 1.5 and 1.51 mC before and after electrolysis, respectively).

When the activity for both OER and ORR was normalized for the amount of electroactive Fe, the electrochemical responses of differently loaded Fe(np)/nC electrodes converge basically to the same behaviour (see Figure 16).

The good accessibility of the iron nanoparticles surface for electrocatalytic reactions is particularly appreciated when comparing the charge of iron reduction/oxidation to the overall loading of iron on the studied electrodes, i.e. the fraction of iron that is electrochemically active. Considering a mean iron content of 2.6 at %, the fraction of iron that is accessible by the solution and available for electrochemical reactions is in the order of 20-40% depending on the catalyst loading (Table 4). These are very high values (10-20 % are common values for commercial Pt/C 20% wt. catalysts) and reveal the optimal homogeneity and iron nanoparticles distribution in Fe(np)/nCs.

The interesting catalytic behavior of these specific composite materials observed during the TGA measurements together with the high fraction of electrochemically active iron, prompted us to carefully evaluate their characteristics in terms of the OER and ORR with an electrochemical setup. From the electrochemical characterization, the composite Fe(np)/nCs is revealed to be an efficient catalyst for the ORR in alkaline solution.

The overpotential (η) of ~540 mV required to reach a current density of 1 mA/$cm^2$ is very similar to that of other Fe-based electrocatalysts, that however are less active than MnOx, the most promising earth-abundant ORR catalyst. The selectivity of Fe(np)/nCs towards the reduction of oxygen gas to water (to hydroxide ions due to the pH) at all catalyst loading (Figure 14) is remarkable, as evidenced by the CVs at different rotation speed and the following Koutecky-Levich analysis. The Koutecky-Levich analysis is a common method for the evaluation of ORR kinetics, particularly useful to get information on the reaction mechanism and to discriminate which among the two-electrons (from $O_2$ to $H_2O_2$) or the four-electrons (from $O_2$ to $H_2O$) pathway prevalently occurs. Indeed, $H_2O$ or $H_2O_2$ can be produced following two reaction pathways:

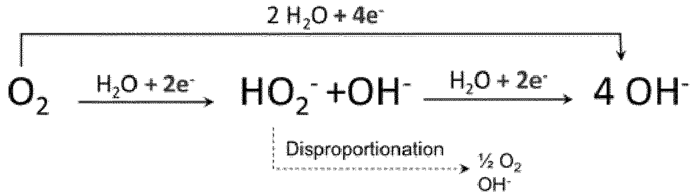


Eq. 2

The method allows extracting ORR kinetics applying ohmic drop and mass transport corrections by using the Levich equation for the convective transport in an RDE configuration (Eq. 2). Previous reports showed a dependence of ORR kinetics on the electrode thickness using Fe-N-C catalysts. An apparent 4-electrons reduction process was indeed observed in thick electrodes, where the fast chemical disproportionation of the produced $H_2O_2$ would occur, then affecting the K-L analysis. However, such effect is excluded in the present case for the low volume of deposited catalyst that corresponds in the worst scenario (loading of 350 $\mu g/cm^2$) to a film thickness in the range of 1.5-2 $\mu m$. For the intermediate loading of Fe(np)/nCs (140 $\mu g/cm^2$), this corresponds to a loading of 16 $\mu gFe/cm^2$. Such iron content is even lower to those commonly used in commercial ORR catalysts (28 $\mu gPt/cm^2$), which in addition contain a noble metal like Pt.

The Tafel analysis highlighted in Figure 6 reveals a slope of 60-62 mV/dec, indication of a good control over $O_2$ mass transport. As previously mentioned, some artefacts on ORR kinetics have been noticed on thick-film electrodes, and the related Tafel slopes values scattered in a wide range of values for a given catalyst. The same slopes found here at different catalyst loading again exclude the occurrence of such artefacts.

Regarding OER electrocatalytic activity, the absolute overpotential required by Fe(np)/nCs to drive OER is remarkably low. To the best of our knowledge this is the highest OER activity so far reported using electrocatalysts entirely Fe-based. We attribute this unusual activity to the very small particle size (2-5 nm) that the method of the invention allows to obtain. Several previous works have focused on bulk or electrodeposited Fe, on pyrolysis of appropriate iron precursors, or on solvothermal approaches. All of these methods produce iron/iron oxide nanoparticles with a much higher 3D dimensionality, that would account for the different observed catalytic activity.

The electroactivity of Fe(np)/nCs for OER in alkaline solution is particularly compelling at all catalyst loadings. Indeed, within the investigated time-scale the overpotential of 0.4 V needed to reach 10 $mA/cm^2$ is almost identical to that of state-of-the-art $RuO_2$, $IrO_2$ and $LiCoO_2$ catalysts ($\eta$= 0.38-0.39 V). In the case of OER, the observed low Tafel slopes of ~ 40 mV/dec (Figure 6a) are not exclusive and characteristic of a single rate determining step but can be associated to different reaction mechanisms depending especially on the surface coverage of adsorbed intermediates. In general, Tafel slopes <120 mV/dec indicate how the formation of the first adsorbed intermediate (Eq. 3) is not limiting OER on Fe(np)/nC.

$$M + OH^- = MOH + e- \qquad \text{Eq. 3}$$

The observed activity of the different Fe(np)/nCs electrodes for ORR and OER are summarized in the Tafel plot of Figure 6a, which provide information about the underlying reaction mechanisms. From the analysis of the Tafel plot, is of great interest to note the bi-functional behaviour of Fe(np)/nC, for both ORR and OER. As mentioned, the electroactivity for the latter reaction is particularly compelling at all catalyst loadings, whereas the driving force for ORR is somewhat larger, but in the range of other reported Fe-based, cheap and earth-abundant catalysts. Overall, the total overpotential required to reach the benchmark values of 10 $mA/cm^2$ current density for both reactions is in the order of 1 V and is in line with the state-of-the-art bi-functional materials. Notice that, the top efficient electrocatalysts, are more precious and are commonly tested at higher catalyst loadings (Fe-N-C/NiFe-LDH, IrO2, Pt/C). This peculiarity can be seen in Figure 6b, where a direct comparison with some of the best-performing bi-functional catalysts reveals how Fe(np)/nC is useful for oxygen electrocatalysis, especially when all its compelling aspects like the very low specific Fe loading, the high-quality of the carbon support, stability and low cost are considered. In particular, Fe(np)/nC stands out as OER catalyst, and having the advantages to be cheap and free of high-cost and time consuming purification procedures (i.e. calcination) its value may outperform that of actual precious catalysts. In contrast to various literature examples, where nitrogen doping of the carbon framework is essential for good electroactive activity, the presented electro catalytic properties of Fe(np)/nC composite material is observable without any nitrogen doping of the carbon framework. It is noteworthy, that the Fe(np)/nC is truly a sustainable catalytic system, because iron oxides are used as catalytic active centers, the nanographite is prepared from renewable resources.

Accordingly, this example clearly demonstrates that examples of nanographene-supported metal and/or metal oxide nanoparticle composites exhibit ORR and OER bi-functional activity and have remarkable electro catalytic properties.

In particular, this example clearly demonstrates that the composite obtained by the method of the present

invention is useful for example as catalytic material, as electrode material for use in electrocatalysis, for example in fuel cell and energy storage technology, as electrocatalyst for oxygen reduction reaction (ORR) or oxygen evolution reaction (OER) and as bifunctional electrocatalyst for oxygen reduction reaction (ORR) or oxygen evolution reaction (OER).

This is particularly important in light of the fact that development of sustainable materials capable of meeting the demand of high performance devices such as energy storage systems (e.g. fuel cells) or catalysts for chemical reactions is still a major challenge. In the context of energy storage and conversion, oxygen electrocatalysis has a dramatic influence on the energy efficiency of the technology developed so far. As a matter of fact, oxygen reduction reaction (ORR: $O_2 + 2H_2O + 4e^- \rightarrow 4OH^-$) is a key reaction for low temperature hydrogen fuel-cell technology, both in acidic and alkaline environments. The reaction requires an efficient 4-electron reduction of oxygen, and catalysts, selectively generating water are highly sought after. On the other hand, the oxygen evolution reaction (OER: $4OH^- \rightarrow O_2 + 2H_2O + 4e^-$) is central in respect to the electrolysis of water, the development of rechargeable air batteries and artificial photosynthesis. Both reactions, however, have quite large kinetic limitations that limit the energy efficiency of the above-mentioned technologies. Bi-functional catalysts that combine both OER and ORR are thus highly sought after for various applications. Platinum group metals are among the best catalysts for these electro-catalytic reactions, however their scarcity and high cost call for alternatives. Earth-abundant catalysts are sought as promising options, but their development is still a demanding technological hurdle. Major difficulties are related to their lower intrinsic catalytic activity and additional issues such as limitation in mass transport, number of active sites, and chemical stability. Remarkable improvements have been obtained by nanostructuring of earth-abundant metals. In this context, a defined synthesis is crucial to generate materials with specific properties for catalytic purposes, improving the overall performance of catalysts, their selectivity and their durability. Thus, the composites according to the present invention fills a real need for bifunctional catalysts, in particular that could catalyze both OER and ORR.

Spectroscopic analyses:

Raman spectroscopy. Raman spectroscopic characterization was carried out on a Horiba Jobin Yvon Xplora microscope equipped with a cooled Andor CCD detector; excitation wavelength: 532 nm, calibration on HOPG, laser spot size of ~1 μm, 1200 lines per mm grating, objective Olympus 50x LWD. Raman mappings were performed with a motorized x-y μm scanning table. 1681 individual spectra were recorded on each sample. Calculations were performed using Labspec6 and Origin 9.2.

X-ray powder diffraction (XRD): XRD patterns were collected on a PANalitycal X'pert MPD-PRO Bragg-Brentano θ-θ geometry diffractometer equipped with a secondary monochromator over an angular range of 2θ = 8-80°. Each acquisition lasted for 2 hours and 5 minutes. The Cu-Kα radiation was generated at 45 KV and 40 mA (λ= 0.15418 nm). The samples were put on silicon wafer ("zero background") sample holder and flattened with a piece of glass.

Thermo gravimetric characterization (TGA): Thermo gravimetric characterization was performed on a TA Q50 TGA. Between 4-7 mg of composite sample was weighed in a Pt-crucible. The measurements were performed under air between 30-750°C with a heating slope of 10°C/min.

X-ray photoelectron spectroscopy (XPS): A ThermoFisher Scientific K-ALPHA spectrometer was used for surface analysis with a monochromatized AlKα source (hν = 1486.6 eV) and a 200 microns spot size. A pressure of $10^{-7}$ Pa was maintained in the chamber during analysis. The full spectra (0-1150 eV) were obtained with constant pass energy of 200 eV and high resolution spectra at constant pass energy of 40 eV. Charge neutralization was applied for all samples. High resolution spectra were fitted and quantified using the AVANTAGE software provided by ThermoFisher Scientific and the Scofield sensitivity factors available from the internal database.

Atomic force microscope (AFM): AFM images in ambient air were acquired using a Nanoscope III microscope operated in tapping mode using 8 nm radius tips MPP-111000.

Scanning tunneling microscope (STM): Ambient STM images were recorded on Nanoscope III microscope operated in STM mode using freshly cut platinum/iridium wire (Pt 80/Ir 20, 0.25 mm diameter).

Deposition of the solutions onto substrates for AFM and STM imaging: Deposits of the nanographene composite dispersions were prepared by drop-casting the dispersion directly onto freshly cleaved mica or HOPG substrates. In detail, 20μl of composite dispersions in THF were deposited on ca 1 $cm^2$ substrates. The coated surfaces were dried under vacuum at room temperature and then washed carefully using deionized water. Finally, the coated mica substrates were dried at 100 °C; the HOPG substrate was dried at 50 °C under vacuum overnight.

Transmission electron microcopy (TEM): TEM measurements were performed on a TEM-FEG HR (JEOL 2200FS). TEM grids have been prepared by drop casting 20 μl of nanocomposite dispersion in THF directly onto a SF400-CU TEM grid (Electron microscopy science)

**Comparative Example: Preparation of large flake graphene-supported metal and/or metal oxide nanoparticle composites**

A composite material was prepared using natural graphite as starting material in the synthesis, instead of nanographite. The experimental procedure has been carried out according to the procedure of Example 1, namely:

- graphite intercalation compound (GIC) was prepared by reduction of natural grahite with cleaned potassium (stoichiometry $KC_8$), under the same reaction conditions as Example 1b;
- the resulting GIC was dissolved in absolute THF to lead to a graphenide solution in THF, under the same reaction conditions as Example 1c; and
- the resulting graphenide solution was reacted with recrystallized iron tetrafluoroborate salt dissolved in THF, under the same reaction conditions as Example 1d.

The resulting composite product has been characterised by the means of TEM analysis (Figure 17). In the image, it becomes apparent that particle formation of iron oxide can also be found when natural graphite is used as starting material. However, the size of the particles is significantly larger than what was observed in the case of nanographite (Example 1). Specifically, in this comparative Example, the iron oxide particle size is in the range of 20-50 nm, which is about an order of magnitude higher than that obtained with nanographene-supported metal and/or metal oxide nanoparticle composites (typically 2-5 nm, Example 1).

**LIST OF REFERENCES**

[1] Hof et al. "Conductive inks of graphitic nanoparticles from a sustainable carbon feedstock" Carbon 111 (2017) 142-149.

[2] A. Herold, "Synthesis of graphite intercalation compounds", in Chemical physics of intercalation, A.P. Legrand and S. Flandrois Eds, NATO ASI Series, series B, Vol. 172, pp. 3-45 (1987).

[3] WO 2009/056696

[4] C. Stein, J. Poulenard, L. Bonnetain, J. Golé, C.R. Acad. Sci. Paris 260, 4503 (1965).

[5] F. Béguin and R. Setton New ternary lamellar compounds of graphite, Carbon 13, 293-295 (1975).

[6] Hodge, S. A., Fogden, S., Howard, C. A., Skipper, N. T. & Shaffer, M. S. P. Electrochemical Processing of Discrete Single-Walled Carbon Nanotube Anions. ACS Nano 7, 1769-1778 (2013). [27]

[7] Haeshin Lee et al. "Facile Conjugation of Biomolecules onto Surfaces via Mussel Adhesive Protein Inspired Coatings Adv Mater. 2009 Jan 26; 21(4): 431-434.

**Claims**

**1.** A method for preparing nanographene-supported metal and/or metal oxide nanoparticle composites, comprising the following steps:

a) dissolving a nanographite intercalation compound in an aprotic organic solvent (A) or a mixture (A') of aprotic organic solvents under anhydrous inert atmosphere, preferably in the absence of sonication; thereby leading to an organic nanographenide solution;
b) reacting the organic nanographenide solution obtained in step a) with a suitable amount of at least one metal salt and/or at least one metal complex or a mixture of at least two metal salts and/or metal complexes under anhydrous inert atmosphere; thereby leading to a suspension of nanographene-supported metal nanoparticles; and
c) optionally oxidizing the metal nanoparticles present on the nanographene support;

wherein the process is free of reducing agent other than the nanographenide formed in step a).

2. A method according to claim 1, wherein the nanographite intercalation compound is prepared by (i) reduction of nanographite by an alkali metal $M_0$ in vapour phase; (ii) electrochemical reduction of nanographite; or (iii) reduction of nanographite by an alkali metal salt of formula $M_0^+B^-$, wherein $M_0^+$ represents an alkali metal cation wherein the alkali metal is preferably selected from lithium, sodium, potassium, rubidium or cesium, more preferably lithium, sodium or potassium; and $B^-$ represents an anion of an organic radical;
wherein the nanographite preferably has a lateral size between 1 and 900 nm, preferentially between 1 and 200 nm, preferentially between 1 and 100 nm, preferentially between 1 and 25 nm.

3. A method according to claim 1 or 2, wherein the oxidizing step c) is carried out by exposing the suspension obtained in step b) to air and/or water and/or another oxidation agent.

4. A method according to any one of claims 1 to 3, wherein the aprotic organic solvent (A) is selected from ethers such as tetrahydrofuran (THF), methyl-THF (Me-THF), dimethoxyethane (DME), methyl tert-butyl ether (MTBE), diethyl ether, or CycloPentylMethylEther (CPME); dimethylsulfoxide (DMSO); N-methylpyrrolidone (NMP); dimethylformamide (DMF); N-methylformamide (NMF); sulfolane; acetonitrile; nitromethane; ethylacetate; 2-butanone; or dimethylacetamide (DMA); and mixture (A') is selected from mixtures of two or more of these.

5. A method according to any one of claims 1 to 4, wherein a single aprotic organic solvent is used in step a), preferably an ether (such as THF), DMA or DMF, most preferably THF.

6. A method according to any one of claims 1 to 5, wherein, in the suspension of nanographene-supported metal nanoparticles obtained in step b), the metal nanoparticles are metal alloy nanoparticles.

7. A method according to claim 6, wherein the metal alloy is selected from Fe-Pt, Fe-Rh, Co-Pt or Co-Rh alloy.

8. A method according to any one of claims 1 to 5, wherein, in the at least one metal salt and/or at least one metal complex or a mixture of at least two metal salts and/or metal complexes of step b), the metal is selected from lanthanides, actinides, transition metals, and/or post-transition metals; preferably transition metals such as Fe, Ni, Pt, Rh, Mn, Cu and/or Co.

9. A method according to any one of claims 1 to 5, wherein the at least one metal salt and/or at least one metal complex or a mixture of at least two metal salts and/or metal complexes used in step a) is selected from:

(i) Fe, Ni, Co, Cu, and/or Mn salts of Cl, Br, I, OTF, $BF_4$, $PF_6$, acetate-, acetylacetonate- or [(bis(trifluoromethylsulfonyl)amide)]; or
(ii) a mixture of:

- at least one Fe and/or Co salt of Cl, Br, I, OTF, $BF_4$, $PF_6$, acetate-, acetylacetonate- or [(bis(trifluoromethylsulfonyl)amide)]; and
- at least one Pt and/or Rh salt of Cl, Br, I, OTF, $BF_4$, $PF_6$, acetate-, acetylacetonate- or [(bis(trifluoromethylsulfonyl)amide)], for example $PtCl_2$ or $RhCl_3$.

10. A method according to any one of claims 1 to 9, wherein the metal and/or metal oxide nanoparticles formed on the nanographene support, have an average size of 2-5 nm.

11. Nanographene-supported metal and/or metal oxide nanoparticle composite obtained by a method according to any one of claims 1 to 10.

12. Nanographene-supported Fe, Ni and/or Co oxide nanoparticle composite comprising iron oxide, nickel oxide and/or cobalt oxide nanoparticles grafted on nanographene; wherein the nanographene support is mostly (if not only) single-layered nanographene and preferably has a lateral size between 1 and 900 nm, preferentially between 1 and 200 nm, preferentially between 1 and 100 nm, preferentially between 1 and 25 nm; and wherein the composite exhibits ferromagnetic properties.

13. Nanographene-supported Fe/Pt, Fe/Rh, Co/Pt and/or Co/Rh metal alloy nanoparticle composite comprising Fe/Pt, Fe/Rh, Co/Pt and/or Co/Rh metal alloy or metal alloy oxide nanoparticles grafted on nanographene; wherein the nanographene support is mostly (if not only) single-layered nanographene and preferably has a lateral size between 1 and 900 nm, preferentially between 1 and 200 nm, preferentially between 1 and 100 nm, preferentially between

1 and 25 nm; and wherein the composite exhibits magnetic properties.

**14.** Composite according to any one of claims 11 to 13, wherein the metal oxide or metal alloy nanoparticles present on the nanographene support have an average size of 2-5 nm.

**15.** Composite according to any one of claims 11 to 14, wherein one or more biocompatible groups are anchored on the carbon framework of the composite's nanographene support, or on the metal or metal oxide itself, either covalently or non-covalently.

**16.** Use of a composite according to any one of claims 11 to 14:

- as catalytic material;
- as electrode material for use in electrocatalysis, for example for applications in fuel cell and energy storage technology;
- as electrocatalyst for oxygen reduction reaction (ORR) or oxygen evolution reaction (OER);
- as bifunctional electrocatalyst for oxygen reduction reaction (ORR) and oxygen evolution reaction (OER);
- as catalyst for gas-phase chemical reactions, such as hydrogenation of CO in the Fischer-Tropsch process;
- as slurry catalyst for liquid-phase chemical reactions, such as liquid phase oxidation processes (such as oxidations of aldehydes to carboxylic acids, benzyl alcohol to benzaldehyde, vanillyl alcohol to vanillin) or liquid phase reduction reactions; and/or
- for water purification and waste water treatment; for example for removing toxic metals, such as arsenic, from ground water; and/or
- as slurry catalyst for liquid-phase chemical reactions, wherein the metal oxide is a Fe, Ni or Co oxide or a mixture of at least two of them, and the catalyst composite is separated from the reaction mixture by application of an electromagnetic field.

**17.** Composite according to claim 15 for use in the treatment of cancerous tumors by hyperthermia, and/or as contrast agent in Magnetic Resonance Imagery.

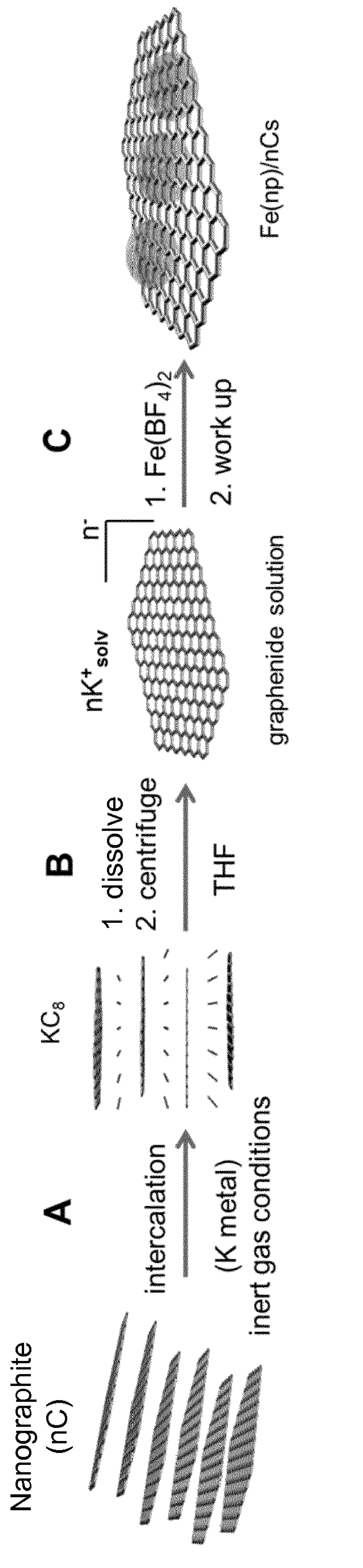
Nanographite
(nC)
A
intercalation
(K metal)
inert gas conditions
$KC_8$
B
1. dissolve
2. centrifuge
THF
$nK^+_{solv}$
$n^-$
graphenide solution
C
1. $Fe(BF_4)_2$
2. work up
Fe(np)/nCs

FIGURE 1

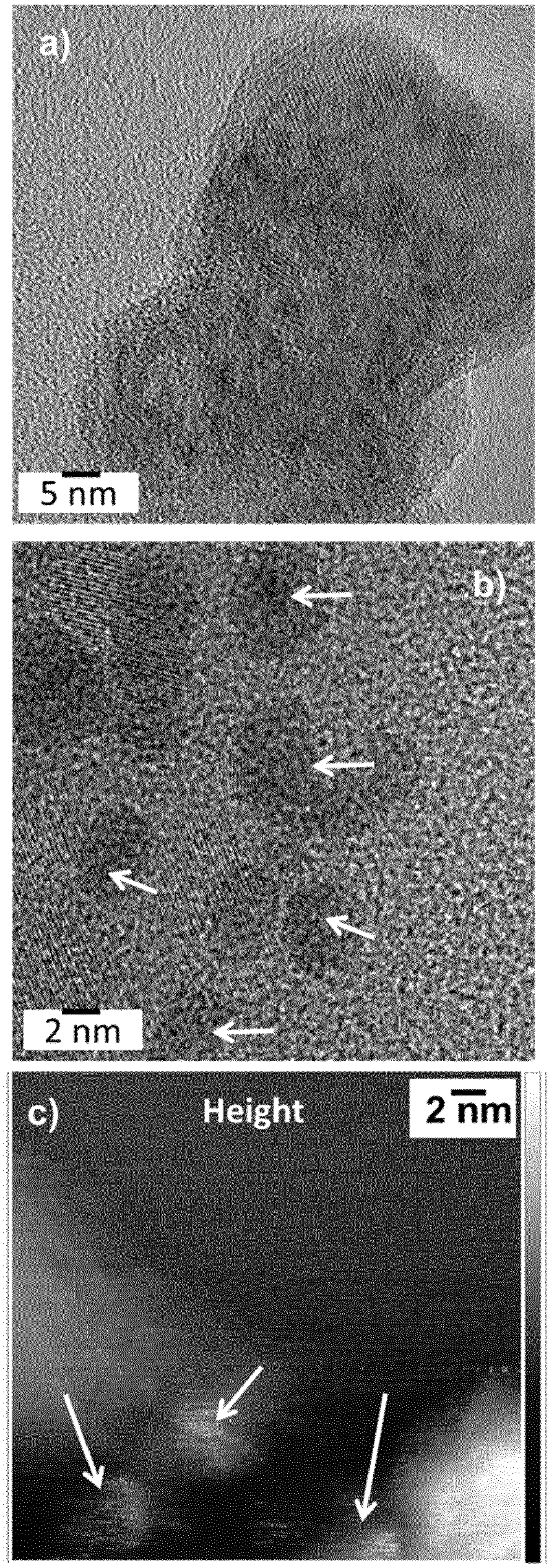


FIGURE 2

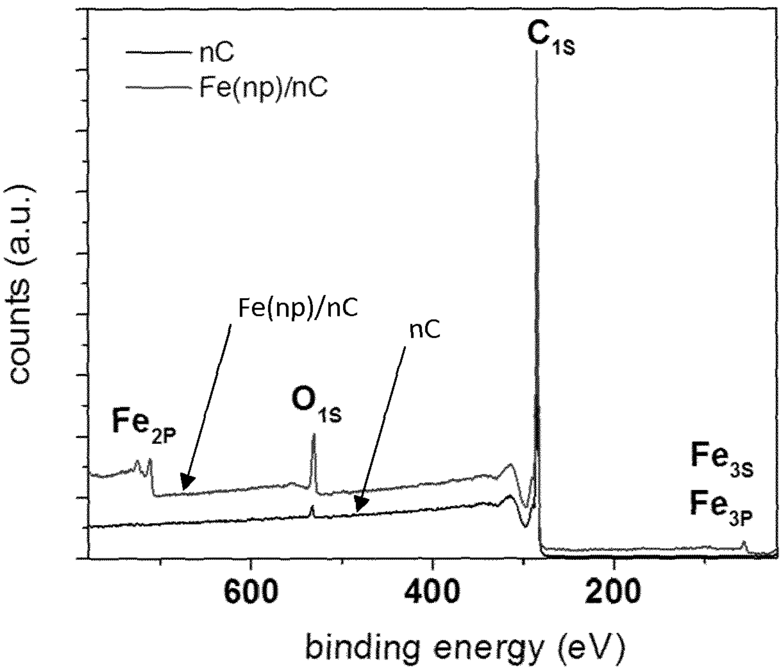
nC
Fe(np)/nC
C1S
counts (a.u.)
Fe(np)/nC
nC
Fe2P
O1S
Fe3S
Fe3P
600
400
200
binding energy (eV)

FIGURE 3

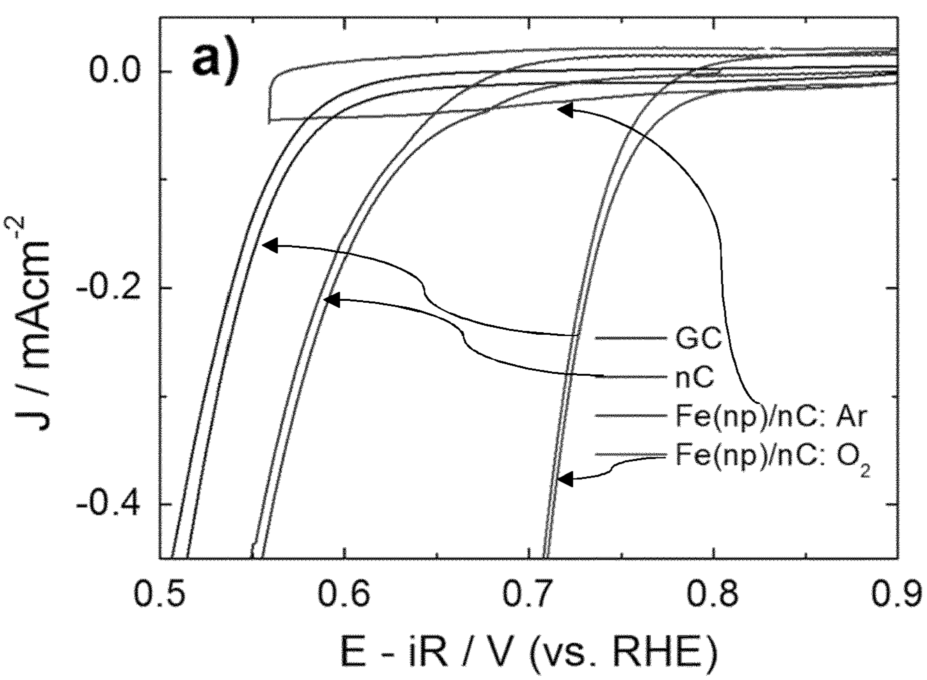
a)
0.0
-0.2
-0.4
J / mAcm-2
0.5
0.6
0.7
0.8
0.9
E - iR / V (vs. RHE)
GC
nC
Fe(np)/nC: Ar
Fe(np)/nC: O2

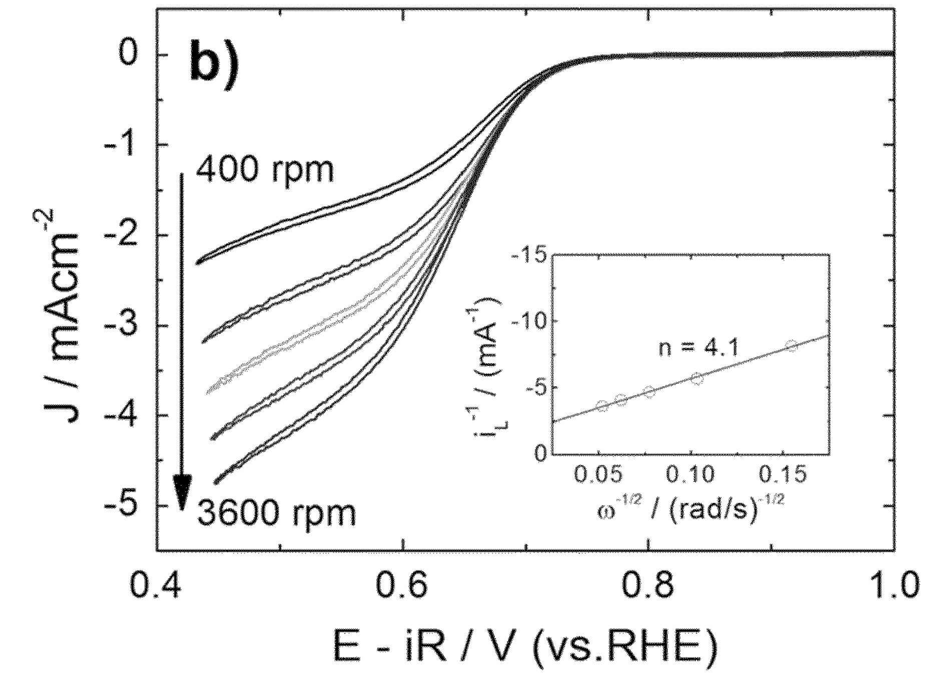
b)
0
-1
-2
-3
-4
-5
J / mAcm-2
400 rpm
3600 rpm
0.4
0.6
0.8
1.0
E - iR / V (vs.RHE)
-15
-10
-5
0
n = 4.1
iL-1 / (mA-1)
0.05
0.10
0.15
ω-1/2 / (rad/s)-1/2

**FIGURE 4**

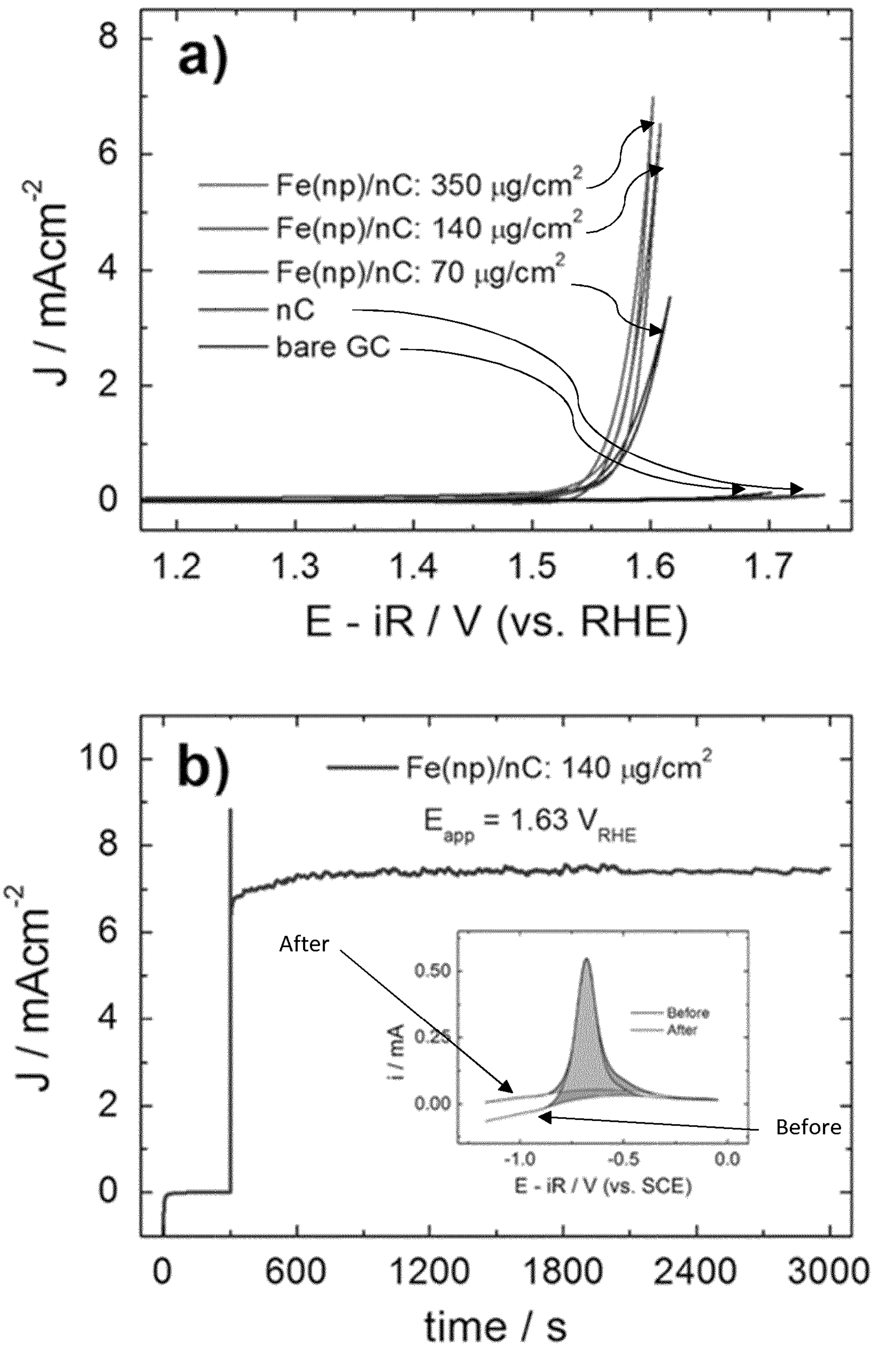
a)
J / mAcm-2
E - iR / V (vs. RHE)
Fe(np)/nC: 350 μg/cm2
Fe(np)/nC: 140 μg/cm2
Fe(np)/nC: 70 μg/cm2
nC
bare GC
b)
Fe(np)/nC: 140 μg/cm2
Eapp = 1.63 VRHE
time / s
After
Before
i / mA
E - iR / V (vs. SCE)

**FIGURE 5**

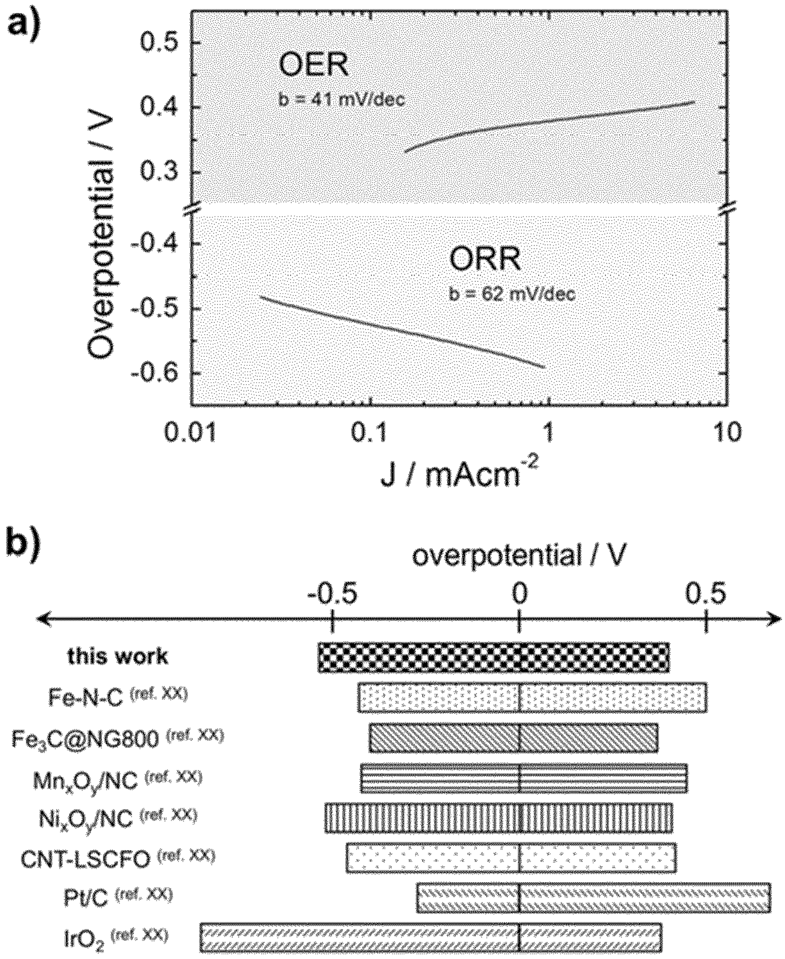
a)
OER
b = 41 mV/dec
ORR
b = 62 mV/dec
Overpotential / V
0.5
0.4
0.3
-0.4
-0.5
-0.6
0.01
0.1
1
10
J / mAcm-2
b)
overpotential / V
-0.5
0
0.5
this work
Fe-N-C (ref. XX)
Fe3C@NG800 (ref. XX)
MnxOy/NC (ref. XX)
NixOy/NC (ref. XX)
CNT-LSCFO (ref. XX)
Pt/C (ref. XX)
IrO2 (ref. XX)

**FIGURE 6**

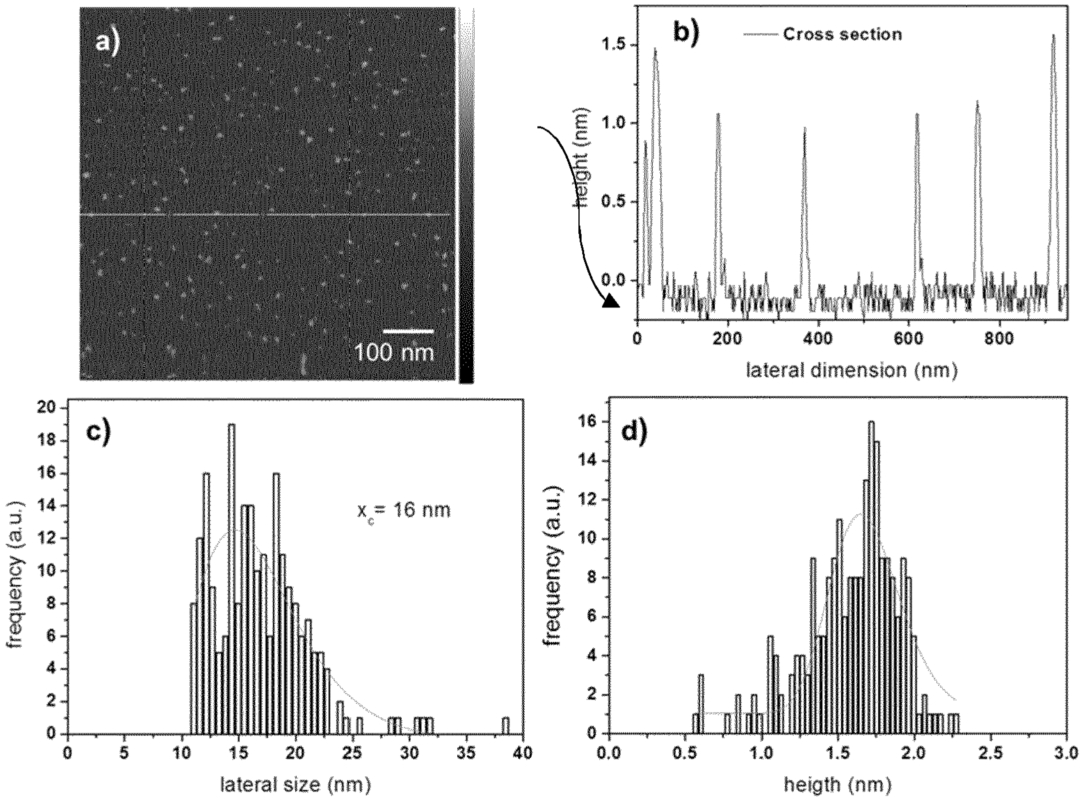


FIGURE 7

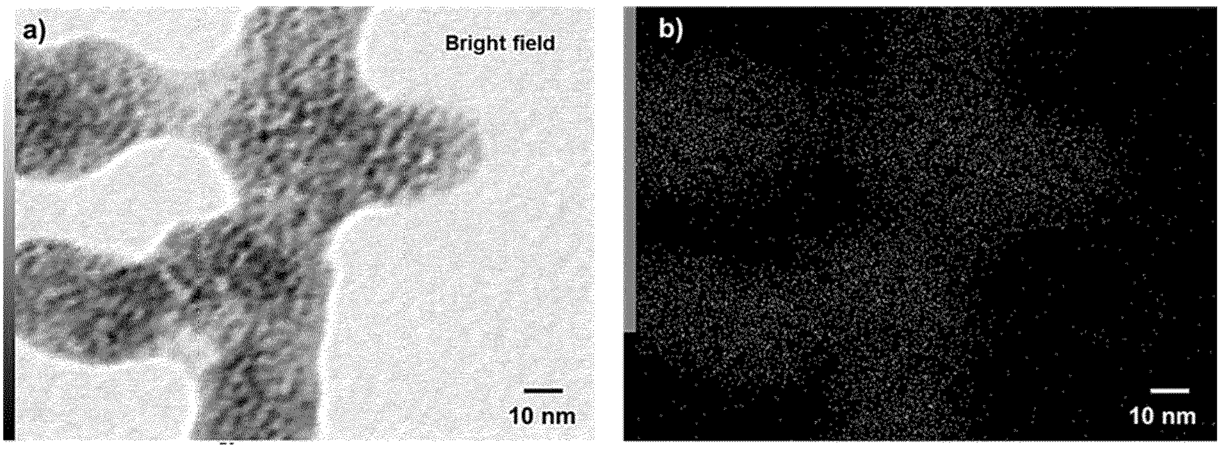


FIGURE 8

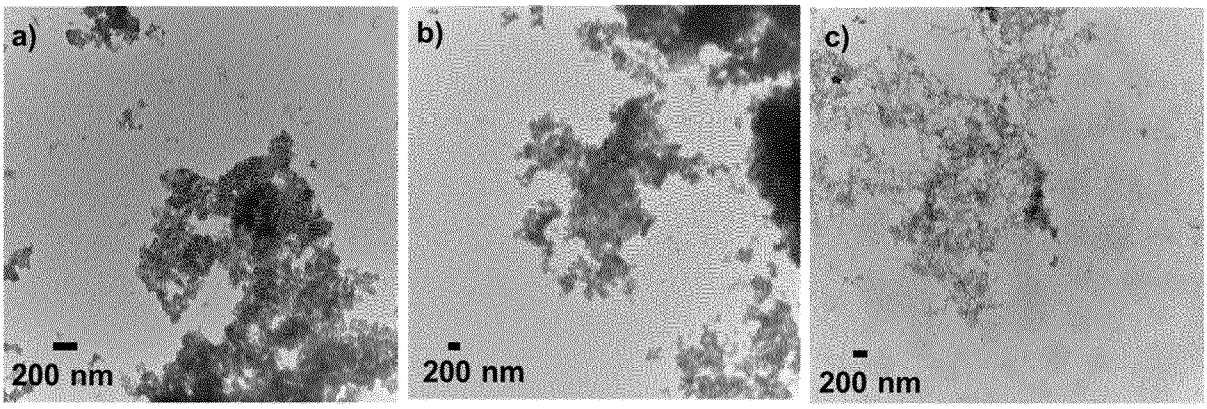


FIGURE 9

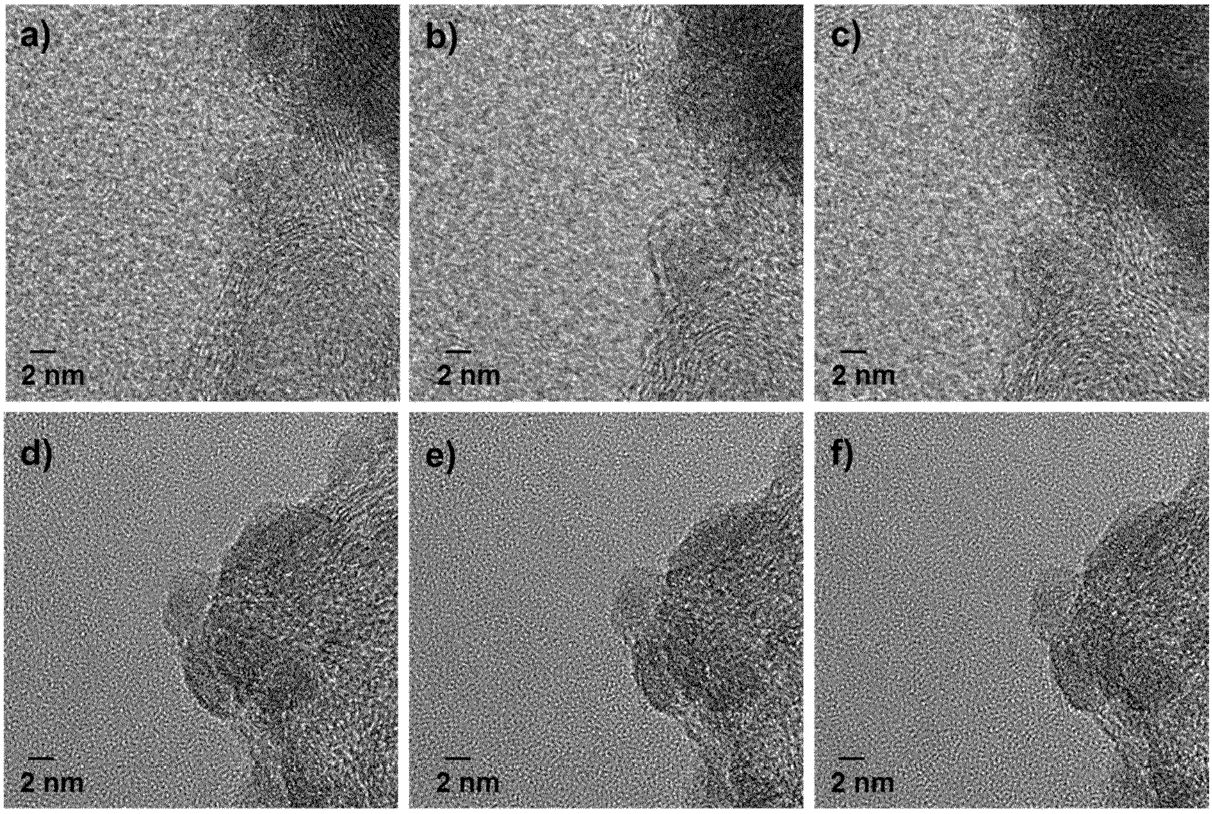


FIGURE 10

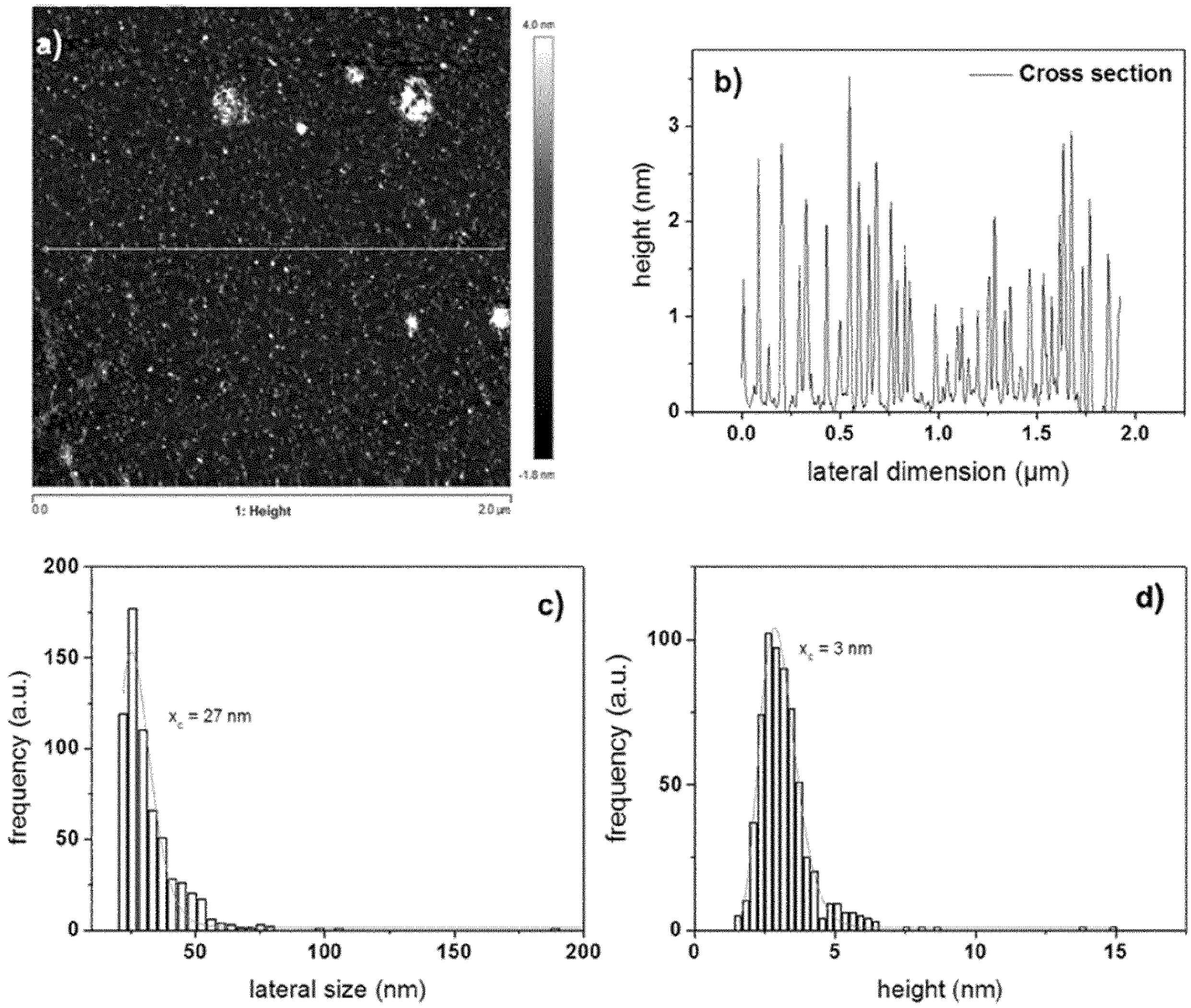
a)
4.0 nm
-1.8 nm
0.0
1: Height
2.0 µm
b)
Cross section
height (nm)
lateral dimension (µm)
c)
$x_c$ = 27 nm
frequency (a.u.)
lateral size (nm)
d)
$x_c$ = 3 nm
frequency (a.u.)
height (nm)

FIGURE 11

a)

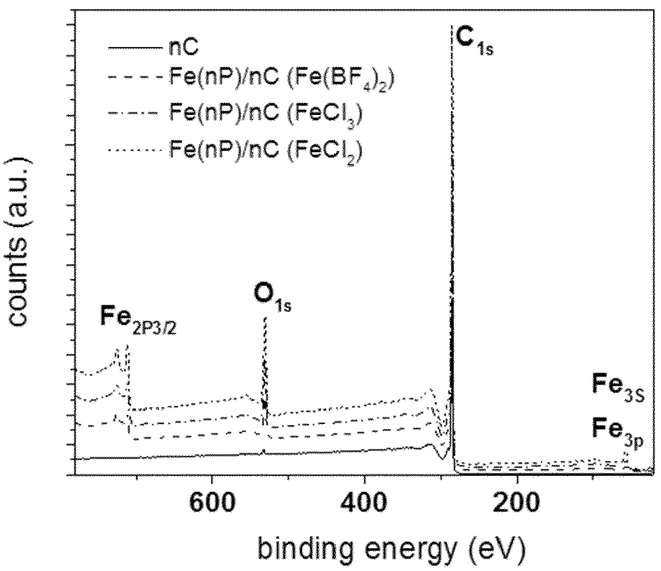
nC
Fe(nP)/nC (Fe(BF4)2)
Fe(nP)/nC (FeCl3)
Fe(nP)/nC (FeCl2)
C1s
O1s
Fe2P3/2
Fe3S
Fe3p
counts (a.u.)
600
400
200
binding energy (eV)

b)

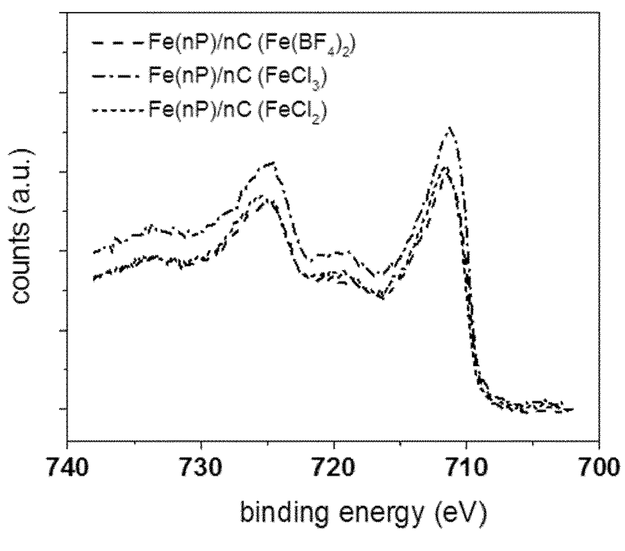
Fe(nP)/nC (Fe(BF4)2)
Fe(nP)/nC (FeCl3)
Fe(nP)/nC (FeCl2)
counts (a.u.)
740
730
720
710
700
binding energy (eV)

**FIGURE 12**

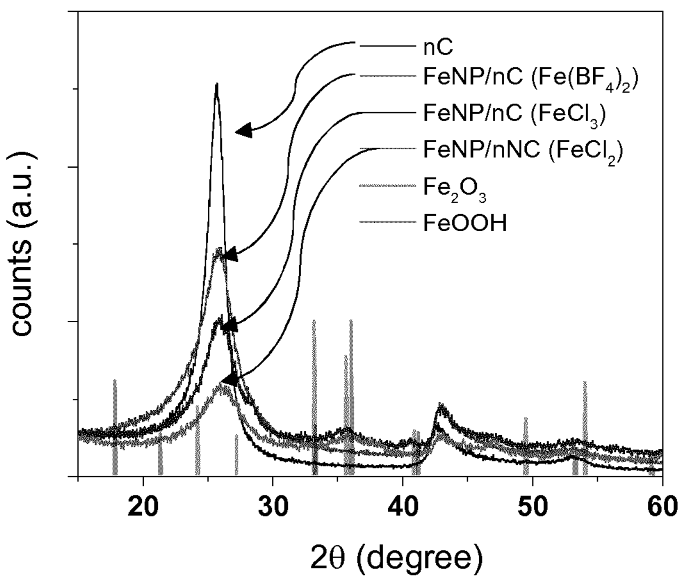


FIGURE 13

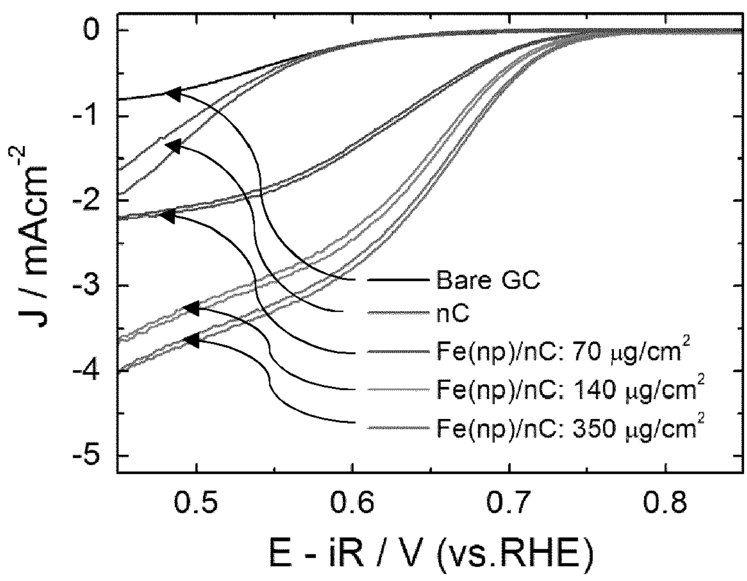


FIGURE 14

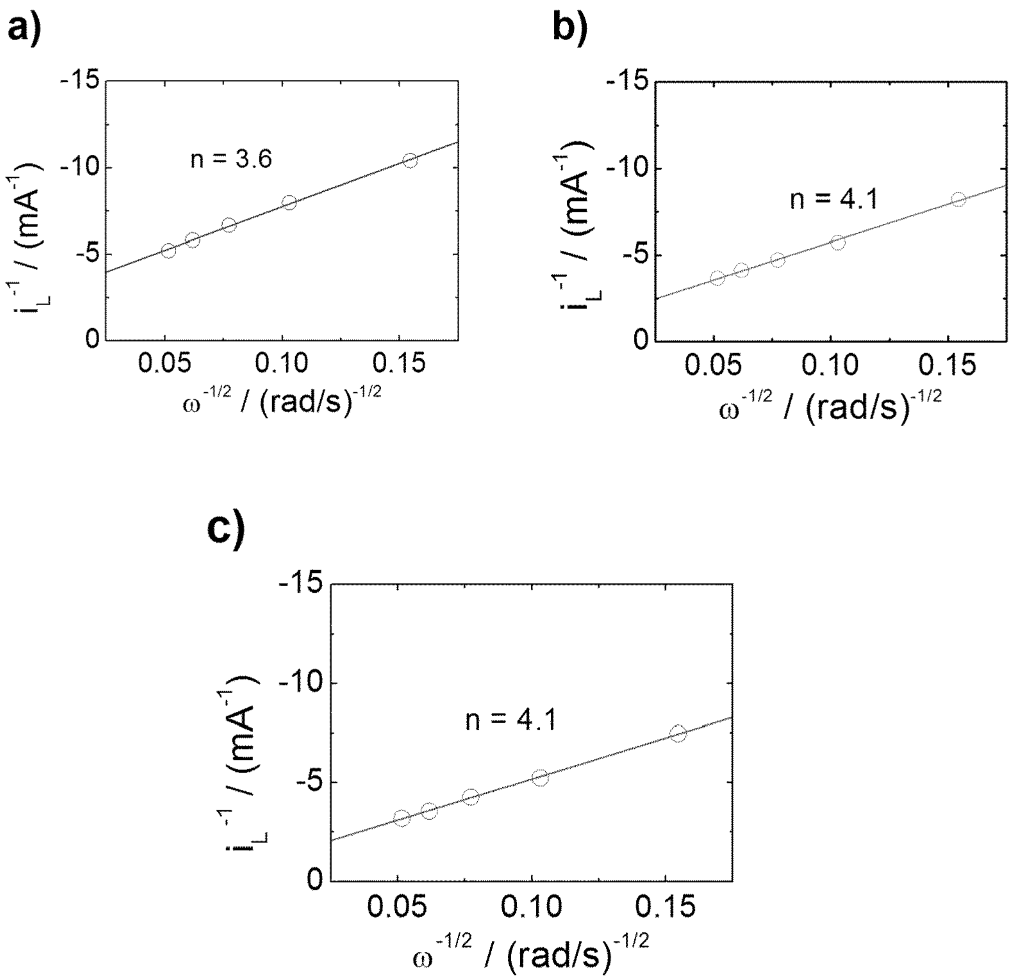
a)
-15
-10
-5
0
n = 3.6
$i_L^{-1}$ / ($mA^{-1}$)
0.05
0.10
0.15
$\omega^{-1/2}$ / (rad/s)$^{-1/2}$
b)
-15
-10
-5
0
n = 4.1
$i_L^{-1}$ / ($mA^{-1}$)
0.05
0.10
0.15
$\omega^{-1/2}$ / (rad/s)$^{-1/2}$
c)
-15
-10
-5
0
n = 4.1
$i_L^{-1}$ / ($mA^{-1}$)
0.05
0.10
0.15
$\omega^{-1/2}$ / (rad/s)$^{-1/2}$

**FIGURE 15**

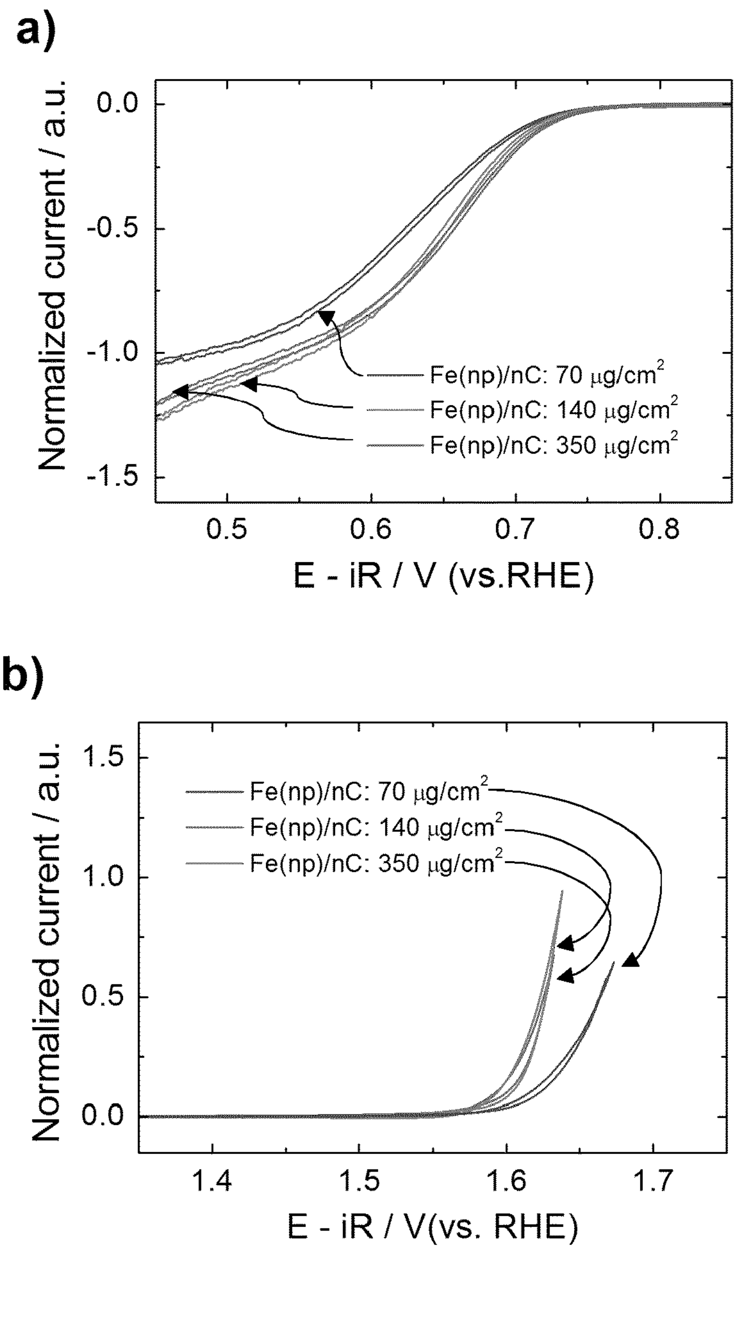
a)
Normalized current / a.u.
0.0
-0.5
-1.0
-1.5
0.5
0.6
0.7
0.8
E - iR / V (vs.RHE)
Fe(np)/nC: 70 μg/cm2
Fe(np)/nC: 140 μg/cm2
Fe(np)/nC: 350 μg/cm2
b)
Normalized current / a.u.
1.5
1.0
0.5
0.0
1.4
1.5
1.6
1.7
E - iR / V(vs. RHE)
Fe(np)/nC: 70 μg/cm2
Fe(np)/nC: 140 μg/cm2
Fe(np)/nC: 350 μg/cm2

FIGURE 16

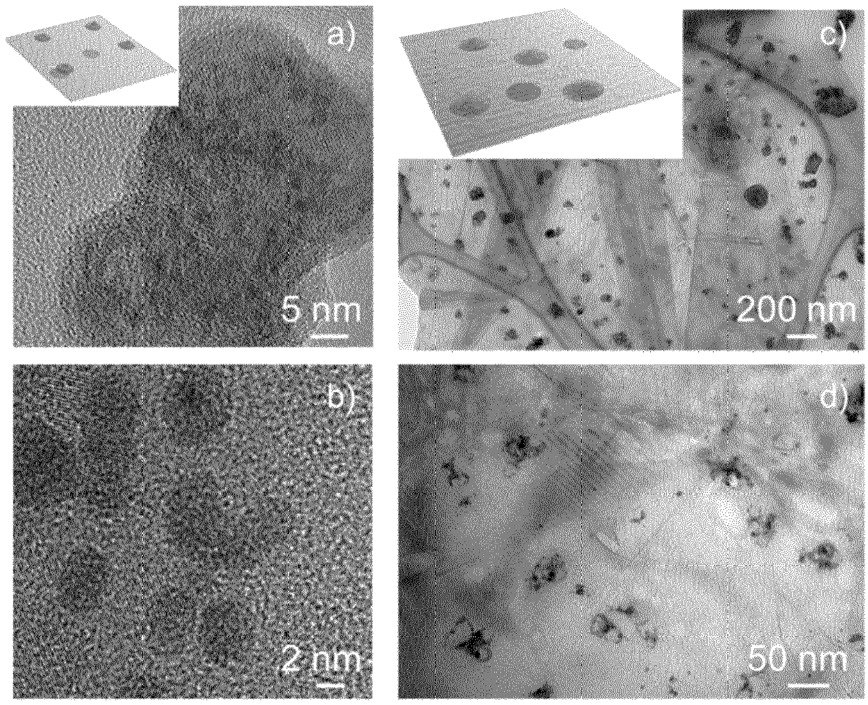


FIGURE 17

Europäisches Patentamt
European Patent Office
Office européen des brevets

# EUROPEAN SEARCH REPORT

Application Number
EP 17 30 5576

| DOCUMENTS CONSIDERED TO BE RELEVANT | | | | |
|---|---|---|---|---|
| Category | Citation of document with indication, where appropriate, of relevant passages | Relevant to claim | CLASSIFICATION OF THE APPLICATION (IPC) |
| T | ALAIN PÉNICAUD ET AL: "Deconstructing Graphite: Graphenide Solutions", ACCOUNTS OF CHEMICAL RESEARCH., vol. 46, no. 1, 15 January 2013 (2013-01-15), pages 129-137, XP055400353, US ISSN: 0001-4842, DOI: 10.1021/ar300141s title; * abstract * * title * * the whole document * ----- | | INV. C01B32/00 B82Y30/00 B82Y40/00 H01B1/04 C01B32/184 C01B32/194 |
| T | ZHONG-SHUAI WU ET AL: "Graphene/metal oxide composite electrode materials for energy storage", NANO ENERGY, vol. 1, no. 1, 2 December 2011 (2011-12-02), pages 107-131, XP055400375, ISSN: 2211-2855, DOI: 10.1016/j.nanoen.2011.11.001 * abstract * * the whole document * * review article on graphene/metal oxide composites * ----- | | TECHNICAL FIELDS SEARCHED (IPC) C01B B82Y H01B |
| X | NEIVA EDUARDO G C ET AL: "Graphene/nickel nanoparticles composites from graphenide solutions", JOURNAL OF COLLOID AND INTERFACE SCIENCE, vol. 453, 30 April 2015 (2015-04-30), pages 28-35, XP029182407, ISSN: 0021-9797, DOI: 10.1016/J.JCIS.2015.04.036 * experimental * * the whole document * ----- | 1-10 | |
| | -/-- | | |

~~The present search report has been drawn up for all claims~~

2

| Place of search | Date of completion of the search | Examiner |
|---|---|---|
| The Hague | 23 August 2017 | Straub, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 30 5576

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/256121 A1 (WOLF AUREL [DE] ET AL) 11 October 2012 (2012-10-11)<br>* paragraphs [0026], [0027]; claims 7,8 *<br>* the whole document *<br>----- | 1-10 | |
| X | WO 2009/143405 A2 (UNIV NORTH CAROLINA [US]; SAMULSKI EDWARD T [US]; SI YONGCHAO [US]; DI) 26 November 2009 (2009-11-26)<br>* claim 28 *<br>* the whole document *<br>----- | 1-10 | |

TECHNICAL FIELDS SEARCHED (IPC)

~~The present search report has been drawn up for all claims~~

2

| Place of search | Date of completion of the search | Examiner |
|---|---|---|
| The Hague | 23 August 2017 | Straub, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 17 30 5576

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-10

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 17 30 5576

The Search Division considers that the present European patent application does not comply with the requirements of unity of invention and relates to several inventions or groups of inventions, namely:

1. claims: 1-10

A method for preparing nanographene-supported metal and/or metal oxide nanoparticle composites, comprising the following steps:
a) dissolving a nanographite intercalation compound in an aprotic organic solvent (A) or a mixture (A') of aprotic organic solvents under anhydrous inert atmosphere, preferably in the absence of sonication; thereby leading to an organic nanographenide solution;
b) reacting the organic nanographenide solution obtained in step a) with a suitable amount of at least one metal salt and/or at least one metal complex or a mixture of at least two metal salts and/or metal complexes under anhydrous inert atmosphere; thereby leading to a suspension of nanographene-supported metal nanoparticles; and
c) optionally oxidizing the metal nanoparticles present on the nanographene support;
wherein the process is free of reducing agent other than the nanographenide formed in step a).

---

2. claims: 11(completely); 14, 15(partially)

Nanographene-supported metal and/or metal oxide nanoparticle composite obtained by a method according to any one of claims 1 to 10.

---

3. claims: 12(completely); 14, 15(partially)

Nanographene-supported Fe, Ni and/or Co oxide nanoparticle composite comprising iron oxide, nickel oxide and/or cobalt oxide nanoparticles grafted on nanographene; wherein the nanographene support is mostly (if not only) single-layered nanographene and preferably has a lateral size between 1 and 900 nm, preferentially between 1 and 200 nm, preferentially between 1 and 100 nm, preferentially between 1 and 25 nm; and wherein the composite exhibits ferromagnetic properties.

---

4. claims: 13(completely); 14, 15(partially)

Nanographene-supported Fe/Pt, Fe/Rh, Co/Pt and/or Co/Rh metal alloy nanoparticle composite comprising Fe/Pt, Fe/Rh, Co/Pt and/or Co/Rh metal alloy or metal alloy oxide nanoparticles grafted on nanographene; wherein the nanographene support is mostly (if not only) single-layered nanographene and preferably has a lateral size between 1 and 900 nm, preferentially between 1 and 200 nm, preferentially between 1 and 100 nm, preferentially between 1 and 25 nm;

Europäisches Patentamt
European Patent Office
Office européen des brevets

## LACK OF UNITY OF INVENTION
## SHEET B

Application Number
EP 17 30 5576

The Search Division considers that the present European patent application does not comply with the requirements of unity of invention and relates to several inventions or groups of inventions, namely:

and wherein the composite exhibits magnetic properties.

---

5. claim: 16(partially)

Use of a composite according to any one of claims 11 to 14:
- as catalytic material

---

6. claim: 16(partially)

Use of a composite according to any one of claims 11 to 14:
- as electrode material for use in electrocatalysis, for example for applications in fuel cell and energy storage technology;

---

7. claim: 16(partially)

Use of a composite according to any one of claims 11 to 14:
- as electrocatalyst for oxygen reduction reaction (ORR) or oxygen evolution reaction (OER);

---

8. claim: 16(partially)

Use of a composite according to any one of claims 11 to 14:
- as bifunctional electrocatalyst for oxygen reduction reaction (ORR) and oxygen evolution reaction (OER);

---

9. claim: 16(partially)

Use of a composite according to any one of claims 11 to 14:
- as catalyst for gas-phase chemical reactions, such as hydrogenation of CO in the Fischer-Tropsch process;

---

10. claim: 16(partially)

Use of a composite according to any one of claims 11 to 14:
- as slurry catalyst for liquid-phase chemical reactions, such as liquid phase oxidation processes (such as oxidations of aldehydes to carboxylic acids, benzyl alcohol to benzaldehyde, vanillyl alcohol to vanillin) or liquid phase reduction reactions;

---

11. claim: 16(partially)

Use of a composite according to any one of claims 11 to 14:

Europäisches Patentamt
European Patent Office
Office européen des brevets

# LACK OF UNITY OF INVENTION SHEET B

Application Number
EP 17 30 5576

The Search Division considers that the present European patent application does not comply with the requirements of unity of invention and relates to several inventions or groups of inventions, namely:

- for water purification and waste water treatment; for example for removing toxic metals, such as arsenic, from ground water;

---

12. claim: 16(partially)

Use of a composite according to any one of claims 11 to 14:
- as slurry catalyst for liquid-phase chemical reactions, wherein the metal oxide is a Fe, Ni or Co oxide or a mixture of at least two of them, and the catalyst composite is separated from the reaction mixture by application of an electromagnetic field.

---

13. claim: 17

Composite according to claim 15 for use in the treatment of cancerous tumors by hyperthermia, and/or as contrast agent in Magnetic Resonance Imagery.

---

## ANNEX TO THE EUROPEAN SEARCH REPORT ON EUROPEAN PATENT APPLICATION NO. EP 17 30 5576

This annex lists the patent family members relating to the patent documents cited in the above-mentioned European search report. The members are as contained in the European Patent Office EDP file on
The European Patent Office is in no way liable for these particulars which are merely given for the purpose of information.

23-08-2017

| Patent document cited in search report | Publication date | Patent family member(s) | Publication date |
|---|---|---|---|
| US 2012256121 A1 | 11-10-2012 | CN 102656114 A | 05-09-2012 |
| | | DE 102009052933 A1 | 19-05-2011 |
| | | EP 2499095 A1 | 19-09-2012 |
| | | JP 2013510787 A | 28-03-2013 |
| | | KR 20120095907 A | 29-08-2012 |
| | | TW 201134758 A | 16-10-2011 |
| | | US 2012256121 A1 | 11-10-2012 |
| | | WO 2011057985 A1 | 19-05-2011 |
| WO 2009143405 A2 | 26-11-2009 | US 2011186789 A1 | 04-08-2011 |
| | | WO 2009143405 A2 | 26-11-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009056696 A


### Non-patent literature cited in the description


- **HOF et al.** Conductive inks of graphitic nanoparticles from a sustainable carbon feedstock. *Carbon,* 2017, vol. 111, 142-149
- Synthesis of graphite intercalation compounds. **A. HEROLD.** Chemical physics of intercalation. 1987, vol. 172, 3-45
- **C. STEIN ; J. POULENARD ; L. BONNETAIN ; J. GOLÉ.** *C.R. Acad. Sci. Paris,* 1965, vol. 260, 4503
- **F. BÉGUIN ; R. SETTON.** New ternary lamellar compounds of graphite. *Carbon,* 1975, vol. 13, 293-295
- **HODGE, S. A. ; FOGDEN, S. ; HOWARD, C. A. ; SKIPPER, N. T. ; SHAFFER, M. S. P.** Electrochemical Processing of Discrete Single-Walled Carbon Nanotube Anions. *ACS Nano,* 2013, vol. 7, 1769-1778
- **HAESHIN LEE et al.** Facile Conjugation of Biomolecules onto Surfaces via Mussel Adhesive Protein Inspired Coatings. *Adv Mater.,* 26 January 2009, vol. 21 (4), 431-434
- **FERDINAND HOF ; KATERINA KAMPIOTI ; KAI HUANG ; CHRISTÈLE JAILLET ; ALAIN DERRÉ ; PHILIPPE POULIN ; HISHAM YUSOF ; THOMAS WHITE ; KRZYSZTOF KOZIOL ; CATHARINA PAUKNER.** Conductive inks of graphitic nanoparticles from a sustainable carbon feedstock. *Carbon,* 2017, vol. 111, 142-149